# FASCICULE DE BREVET EUROPEEN

(11) **EP 0 952 975 B1**
(45) Date de publication et mention de la délivrance du brevet: **10.07.2002**
(21) Numéro de dépôt: 98942768.7
(22) Date de dépôt: 21.08.1998
(51) Int. Cl.: C07C 323/62, C07C 317/44, C07C 391/02, C07D 213/80, C07D 213/79, C07D 213/82, C07D 335/06, A61K 31/19, A61K 31/235, A61K 31/44, A61K 31/095

(54) **COMPOSES BI-AROMATIQUES RELIES PAR UN RADICAL HETEROETHYNYLENE ET COMPOSITIONS PHARMACEUTIQUES ET COSMETIQUES LES CONTENANT**
EINE HETEROETHYNYLEN-BRÜCKE ENTHALTENDE BI-AROMATISCHE VERBINDUNGEN UND DIESE ENTHALTENDE PHARMAZEUTISCHE UND KOSMETISCHE ZUSAMMENSETZUNGEN
BI-AROMATIC COMPOUNDS BOUND BY A HETEROETHYNYLENE RADICAL AND PHARMACEUTICAL AND COSMETIC COMPOSITIONS CONTAINING SAME

(30) Priorité: 21.08.1997 FR 9710554
(43) Date de publication de la demande: 03.11.1999
(73) Titulaire: Galderma Research & Development, 06560 Valbonne (FR)
(72) Inventeur: BERNARDON, Jean-Michel, F-06650 Le Rouret (FR); DIAZ, Philippe, F-06200 Nice (FR)
(74) Mandataire: Tanty, François
(86) Numéro de dépôt international: FR9801835
(87) Numéro de publication internationale: WO9910322

(56) Documents cités:
- EP-A- 0 661 258
- DE-A- 2 130 483
- DATABASE CHEMABS CHEMICAL ABSTRACTS SERVICE, COLUMBUS, OHIO, US STN, accession no. 78:57492, XP002087917 & P. BELTRAME ET AL: J. CHEM. SOC., PERKIN TRANS. 2, no. 1, 1973, pages 63-66,
- DATABASE CHEMABS CHEMICAL ABSTRACTS SERVICE, COLUMBUS, OHIO, US STN, accession no. 101:72030, XP002087918 & V.Z. LATYPOVA ET AL: ZH. OBSHCH. KHIM., vol. 54, no. 4, 1984, pages 848-851,
- K.-L. YU ET AL: BIOORG. MED. CHEM. LETT., vol. 6, no. 23, 1996, pages 2865-2870, XP002057399
- H.SUZUKI ET AL: TETRAHEDRON LETT., vol. 37, no. 21, 1996, pages 3717-3720, XP004029241

## Description

L'invention concerne, à titre de produits industriels nouveaux et utiles, des composés bi-aromatiques dont les noyaux aromatiques sont reliés par un radical divalent hétéroéthynylène. Elle concerne également l'utilisation de ces nouveaux composés dans des compositions pharmaceutiques destinées à un usage en médecine humaine ou vétérinaire, ou bien encore dans des compositions cosmétiques.

Les composés selon l'invention ont une activité marquée dans les domaines de la différenciation et de la prolifération cellulaire et trouvent des applications plus particulièrement dans le traitement topique et systémique des affections dermatologiques liées à un désordre de la kératinisation, des affections dermatologiques (ou autres) à composante inflammatoire et/ou immunoallergique, et des, proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques pour l'hygiène corporelle et capillaire.

Il a déjà été décrit dans l'EP-661 258 des composés bi-aromatiques dont les noyaux aromatiques sont reliés par une liaison divalente propynylène, en tant que substances actives dans des compositions pharmaceutiques ou cosmétiques.

Les composés selon l'EP-661 258 répondent à la formule générale suivante : 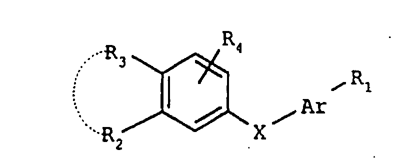 dans laquelle :
Ar est un radical divalent aromatique éventuellement substitué par un radical R₅ ou hétéroaromatique éventuellement substitué par un radical R₆ lorsque l'hétéroatome est l'azote,
R₁ représente H, -CH₃, -CH₂OR₆, -OR₆, -COR₇ ou -S(O)ₜR₉, t étant 0, 1 ou 2,
R₂ et R₃ représentent H, alkyle en C₁-C₂₀, -OR₆ ou -SR₆,
ou R₂ et R₃ pris ensemble forment un cycle à 5 ou 6 chaînons. éventuellement substitué par des groupes méthyles et/ou éventuellement interrompu par un atome d'oxygène ou de soufre,
R₄ et R₅ représentent H, un halogène, alkyle inférieur ou -OR₆,
R₆ représente H, alkyle inférieur ou -COR₉,
R₇ représente H, alkyle inférieur, 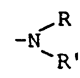 ou -OR₈,
R₈ représente H, alkyle, linéaire ou ramifié, en C₁-C₂₀, alkényle, mono- ou polyhydroxyalkyle, aryle ou aralkyle éventuellement substitué ou un reste de sucre ou d'aminoacide ou de peptide,
R₉ représente alkyle inférieur,
R et R' représentent H, alkyle inférieur, mono ou polyhydroxyalkyle, aryle éventuellement substitué ou un reste de sucre, d'aminoacide ou de peptide ou R et R' pris ensemble forment un hétérocycle, et
X représente un radical divalent qui, de droite à gauche ou inversement, a pour formule : 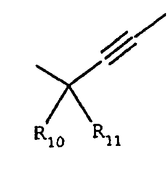
dans laquelle :
R₁₀ représente H, alkyle inférieur ou -OR₆,
R₁₁ représentant -OR₆,
ou R₁₀ et R₁₁ pris ensemble forment un radical oxo (=O),
et les sels desdits composés de formule ci-dessus lorsque R₁ représente une fonction acide carboxylique et les isomères optiques et géométriques de ces dits composés.

Les composés selon la présente invention, par rapport à ceux de l'EP-661 258, se distinguent essentiellement par le fait que le radical X ou radical divalent propynylène a été remplacé par un radical divalent hétéroéthynylène.

D'autres composés bi-aromatiques reliés par une liaison divalente propylène sont connus de K.-L. Yu et al., Bioorg. Med. Chem. lett., 6(23), 2865-2870(1996).

On a en effet constaté de façon inattendue et surprenante que cette modification de structure permettait d'en augmenter de façon significative les propriétés pharmaceutiques et cosmétiques et en outre d'en diminuer certains effets secondaires.

La présente invention a donc pour objet de nouveaux composés qui peuvent être représentés par la formule générale suivante : 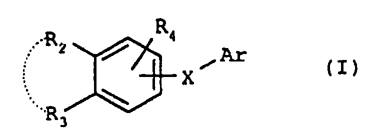 dans laquelle :
Ar représente un radical choisi parmi les formules (a) et (b) suivantes : 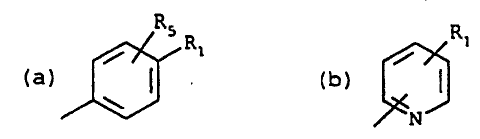
R₁ représente un atome d'halogène -CH₃, -CH₂-OR₇, -OR₇ ou -COR₈,
R₂ et R₃, identiques ou différents, représentent H, alkyle, linéaire ou ramifié, en C₁-C₂₀, cycloalkyle en C₃-C₁₂, ou -OR, l'un au moins de R₂ et R₃ étant alkyle, linéaire ou ramifié, en C₁-C₂₀, ou cycloalkyle en C₃-C₁₀, ou
R₂ et R₃, pris ensemble forment un cycle à 5 ou 6 chaînons, pouvant comporter au moins un méthyle et/ou pouvant être interrompu par un hétéroatome choisi parmi O ou S,
R₄ et R₅ représente H, un atome d'halogène, alkyle, linéaire ou ramifié, en C₁-C₂₀, -OR₇, ou un radical polyéther,
R₇ représente H, alkyle, linéaire ou ramifié en C₁-C₁₀ ou -COR₉,
R₈ représente H, alkyle, linéaire ou ramifié en C₁-C₁₀, -OR₁₀ ou 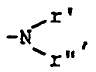
R₉ représente alkyle, linéaire ou ramifié en C₁-C₁₀,
R₁₀ représente H, alkyle, linéaire ou ramifié en C₁-C₂₀, mono- ou polyhydroxyalkyle, allyle, aryle ou aralkyle,
r' et r", identiques ou différents, représentent H, alkyle en C₁-C₁₀, mono- ou polyhydroxyalkyle, aryle, ou pris ensemble avec l'atome d'azote, forment un hétérocycle,
X représente un radical divalent qui de droite à gauche ou inversement a pour formule : 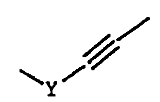
dans laquelle :
Y représente S(O)ₙ ou Se(O)_{n'},
n et n' étant 0, 1 ou 2,
sous réserve que lorsque n=2 et Ar est un radical de formule (a) ci-dessus dans laquelle R₁=-CH₃ et R₅=H, alors l'un des radicaux R₂ ou R₃ est différent de -CH₃,
et les sels des composés de formule (I) lorsque R₁ représente une fonction acide carboxylique ainsi que les isomères optiques desdits composés de formule (I).

Lorsque les composés selon l'invention se présentent sous forme d'un sel, il s'agit de préférence d'un sel d'un métal alcalin ou alcalino-terreux, ou encore de zinc ou d'une amine organique.

Selon la présente invention, on entend par alkyle en C₁-C₁₀, de préférence les radicaux méthyle, éthyle, isopropyle, butyle, tertiobutyle, hexyle, 2-éthyl-hexyle ou octyle.

Par alkyle, linéaire ou ramifié, en C₁-C₂₀, on entend notamment les radicaux méthyle, éthyle, propyle, 2-éthyl-hexyle, octyle, dodécyle, hexadécyle et octadécyle.

Par radical cycloalkyle en C₃-C₁₂ on entend un radical mono ou polycyclique notamment les radicaux cyclopropyle, cyclopentyle, cyclohexyle, 1-méthylcyclohexyle ou 1-adamantyle.

Par radical polyéther, on entend un radical ayant de 2 à 5 atomes de carbone interrompu par au moins deux atomes d'oxygène tels que les radicaux méthoxyméthoxy, méthoxyéthoxy et méthoxyéthoxyméthoxy.

Par monohydroxyalkyle, on entend un radical ayant de préférence 2 ou 3 atomes de carbone, notamment un radical 2-hydroxyéthyle, 2-hydroxypropyle ou 3-hydroxypropyle.

Par polyhydroxyalkyle, on entend un radical ayant de préférence 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par aryle, on entend de préférence un radical phényle éventuellement substitué par au moins un atome d'halogène, un hydroxyle ou une fonction nitro.

Par aralkyle, on entend de préférence le radical benzyle ou phénéthyle éventuellement substitué par au moins un atome d'halogène, un hydroxyle ou une fonction nitro. aminés.

Par hétérocycle, on entend de préférence un radical pipéridino, morpholino, pyrrolidino pipérazino, ou pipérazino substitué en position 4 par un alkyle inférieur en C₁-C₆ ou un mono- ou polyhydroxyalkyle tels que définis ci-dessus.

Lorsque R₁, R₄ et/ou R₅ représente un atome d'halogène, celui-ci est de préférence un atome de fluor, de chlore ou de brome.

Selon une première forme de réalisation préférée, les composés selon l'invention répondent à la formule générale suivante : 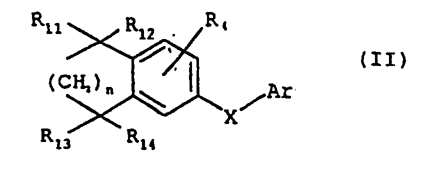 dans laquelle :
Ar représente un radical de formule : 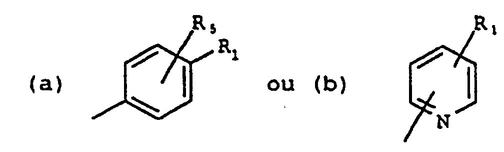
R₁, R₄, R₅ et X étant tels que définis ci-dessus pour la formule (I),
R₁₁, R₁₂, R₁₃ et R₁₄, identiques ou différents représentent H ou -CH₃, et
n est 1 ou 2.

Selon une deuxième forme préférée, les composés selon l'invention répondent à la formule suivante : 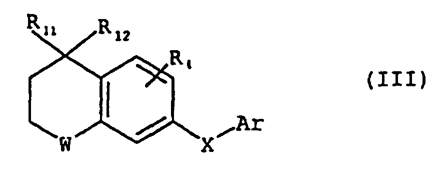 dans laquelle :
W représente S,
R₄, R₁₁, R₁₂, Ar et X étant tels que définis ci-dessus dans les formules (I) et (II).

Enfin, selon une troisième forme préférée, les composés selon l'invention répondent à la formule suivante : 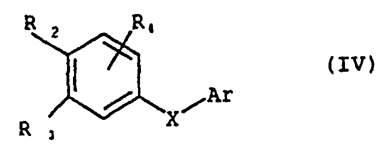 dans laquelle :
R₄, Ar et X sont tels que définis ci-dessus dans les formules (I) à (III), et
l'un des radicaux R₂ et/ou R₃ représente un radical cycloalkyle mono- ou polycyclique en C₅-C₁₀, l'autre représentant l'une des significations données pour R₂ ou R₃.

Parmi les composés des formules (I) à (IV) ci-dessus selon la présente invention, on peut notamment citer les suivants :
- 4-(5,5,8,8-tétraméthyl-5,6,7,8-tétrahydronaphtalèn-2-ylsulfanyléthynyl)benzoate de méthyle,
- acide 4-(5,5,8,8-tétraméthyl-5,6,7,8-tétrahydronaphtalèn-2-ylsulfanyléthynyl)benzoïque,
- 4-(5,5,8,8-tétraméthyl-5,6,7,8-tétrahydronaphtalèn-2-ylsulfonyléthynyl)benzoate de méthyle,
- 4-(5,5,8,8-tétraméthyl-5,6,7,8-tétrahydro-naphtalèn-2-yloxyéthynyl)-benzoate de méthyle,
- acide 4-(5,5,8,8-tétraméthyl-5,6,7,8-tétrahydro-naphtalèn-2-yloxyéthynyl)-benzoïque,
- 4-(5,5,8,8-tétraméthyl-5,6,7,8-tétrahydro-naphtalèn-2-ylsulfanyléthynyl)-benzoate de méthyle,
- acide 4-(5,5,8,8-tétraméthyl-5,6,7,8-tétrahydro-naphtalèn-2-ylsulfanyléthynyl)-benzoïque,
- 4-(5,5,8,8-tétraméthyl-5,6,7,8-tétrahydro-naphtalèn-2-ylsulfonyléthynyl)-benzoate de méthyle,
- acide 4-(5,5,8,8-tétraméthyl-5,6,7,8-tétrahydro-naphtalèn-2-ylsulfonyléthynyl)-benzoïque,
- 4-(5,5,8,8-tétraméthyl-5,6,7,8-tétrahydro-naphtalèn-2-ylsulfinyléthynyl)-benzoate de méthyle,
- acide 4-(5,5,8,8-tétraméthyl-5,6,7,8-tétrahydro-naphtalèn-2-ylsulfinyléthynyl)-benzoïque,
- 4-(5,5,8,8-tétraméthyl-5,6,7,8-tétrahydro-naphtalèn-2-ylsélanyléthynyl)-benzoate de méthyle,
- acide 4-(5,5,8,8-tétraméthyl-5,6,7,8-tétrahydro-naphtalèn-2-ylsélanyléthynyl)-benzoïque,
- 2-hydroxy-4-(5,5,8,8-tétraméthyl-5,6,7,8-tétrahydro-naphtalèn-2-ylsélanyléthynyl)-benzoate de méthyle,
- acide 2-hydroxy-4-(5,5,8,8-tétraméthyl-5,6,7,8-tétrahydro-naphtalèn-2-ylsélanyléthynyl)-benzoïque,
- 6-(4-méthoxyméthoxy-phényléthynylsélanyl)-1,1,4,4-tétraméthyl-1,2,3,4-tétrahydro-naphtalène,
- 6-(5,5,8,8-tétraméthyl-5,6,7,8-tétrahydro-naphtalèn-2-ylsélanyléthynyl)-nicotinate d'éthyle,
- acide 6-(5,5,8,8-tétraméthyl-5,6,7,8-tétrahydro-naphtalèn-2-ylsélanyléthynyl)-nicotinique,
- N-(4-hydroxy-phényl)-4-(5,5,8,8-tétraméthyl-5,6,7,8-tétrahydro-naphtalèn-2-ylsélanyléthynyl)-benzamide,
- 5-(5,5,8,8-tétraméthyl-5,6,7,8-tétrahydro-naphtalèn-2-ylsélanyléthynyl)-pyridine-2-carboxylate de méthyle,
- 2-(4-chloro-phénylsélanyléthynyl)-5,5,8,8-tétraméthyl-5,6,7,8-tétrahydro-naphtalène,
- 4-(3,5,5,8,8-pentaméthyl-5,6,7,8-tétrahydro-naphtalèn-2-ylsélanyléthynyl)-benzoate de méthyle,
- acide 4-(3,5,5,8,8-pentaméthyl-5,6,7,8-tétrahydro-naphtalèn-2-ylsélanyléthynyl)-benzoïque,
- 2-hydroxy-4-(3,5,5,8,8-pentaméthyl-5,6,7,8-tétrahydro-naphtalèn-2-ylsélanyléthynyl)-benzoate de méthyle,
- acide 2-hydroxy-4-(3,5,5,8,8-pentaméthyl 5,6,7,8-tétrahydro-naphtalèn-2-ylsélanyléthynyl)-benzoïque,
- 6-(3,5,5,8,8-pentaméthyl-5,6,7,8-tétrahydro-naphtalèn-2-ylsélanyléthynyl)-nicotinate d'éthyle,
- acide 6-(3,5,5,8,8-pentaméthyl-5,6,7,8-tétrahydro-naphtalèn-2-ylsélanyléthynyl)-nicotinique,
- N-(4-hydroxy-phényl)-6-(3,5,5,8,8-pentaméthyl-5,6,7,8-tétrahydro-naphtalèn-2-ylsélanyléthynyl)-nicotinamidè,
- N-butyl-6-(3,5,5,8,8-pentaméthyl-5,6,7,8-tétrahydro-naphtalèn-2-ylsélanyléthynyl)-nicotinamide,
- morpholin-4-yl-[6-(3,5,5,8,8-pentaméthyl-5,6,7,8-tétrahydro-naphtalèn-2-ylsélanyléthynyl)-pyridin-3-yl]-méthanone,
- 5-(3,5,5,8,8-pentaméthyl-5,6,7,8-tétrahydro-naphtalèn-2-ylsélanyléthynyl)-pyridine-2-carboxylate de méthyle,
- acide 5-(3,5,5,8,8-pentaméthyl-5,6,7,8-tétrahydro-naphtalèn-2-ylsélanyléthynyl)-pyridine-2-carboxylique,
- [4-(5,5,8,8-tétraméthyl-5,6,7,8-tétrahydro-naphtalèn-2-ylsélanyléthynyl)-phényl]-méthanol,
- 4-(5,5,8,8-tétraméthyl-5,6,7,8-tétrahydro-naphtalèn-2-yléthynylsulfanyl)-benzoate de méthyle,
- 4-(5,5,8,8-tétraméthyl-5,6,7,8-tétrahydro-naphtalèn-2-yléthynylsulfonyl)-benzoate de méthyle,
- 4-(5,5,8,8-tétraméthyl-5,6,7,8-tétrahydro-naphtalèn-2-yléthynylsulfinyl)-benzoate de méthyle,
- acide 4-(5,5,8,8-tétraméthyl-5,6,7,8-tétrahydro-naphtalèn-2-yléthynylsulfanyl)-benzoïque,
- acide 4-(5,5,8,8-tétraméthyl-5,6,7,8-tétrahydro-naphtalèn-2-yléthynylsulfonyl)-benzoique,
- acide 4-(5,5,8,8-tétraméthyl-5,6,7,8-tétrahydro-naphtalèn-2-yléthynylsulfinyl)-benzoïque,
- 4-(3,5,5,8,8-pentaméthyl-5,6,7,8-tétrahydro-naphtalèn-2-ylsélanyléthynyl)-phénol,
- 4-(4-hydroxy-5,5,8,8-tétraméthyl-5,6,7,8-tétrahydro-naphtalèn-2-ylsélanyléthynyl)-benzoate d'éthyle,
- 4-(4-méthoxyméthoxy-5,5,8,8-tétraméthyl-5,6,7,8-tétrahydro-naphtalèn-2-ylsélanyléthynyl)-benzoate d'éthyle,
- acide 4-(4-méthoxyméthoxy-5,5,8,8-tétraméthyl-5,6,7,8-tétrahydro-naphtalèn-2-ylsélanyléthynyl)-benzoïque,
- acide 4-(4-pentyloxy-5,5,8,8-tétraméthyl-5,6,7,8-tétrahydro-naphtalèn-2-ylsélanyléthynyl)-benzoïque,
- 4-(3-méthoxyméthoxy-5,5,8,8-tétraméthyl-5,6,7,8-tétrahydro-naphtalèn-2-ylsélanyléthynyl)-benzoate d'éthyle,
- 4-(3-méthoxyéthoxyméthoxy-5,5,8,8-tétraméthyl-5,6,7,8-tétrahydro-naphtalèn-2-ylsélanyléthynyl)-benzoate d'éthyle,
- acide 4-(3-méthoxyéthoxyméthoxy-5,5,8,8-tétraméthyl-5,6,7,8-tétrahydro-naphtalèn-2-ylsélanyléthynyl)-benzoïque,
- acide 4-(3-méthoxyméthoxy-5,5,8,8-tétraméthyl-5,6,7,8-tétrahydro-naphtalèn-2-ylsélanyléthynyl)-benzoïque,
- 4-(3-pentyloxy-5,5,8,8-tétraméthyl-5,6,7,8-tétrahydro-naphtalèn-2-ylsélanyléthynyl)-benzoate d'éthyle,
- acide 4-(3-pentyloxy-5,5,8,8-tétraméthyl-5,6,7,8-tétrahydro-naphtalèn-2-ylsélanyléthynyl)-benzoïque,
- [4-(5,5,8,8-tétraméthyl-5,6,7,8-tétrahydro-naphtalèn-2-ylsélanyléthynyl)-phényl]-carbaldéhyde,
- 4-(4,4-diméthyl-thiochroman-8-ylsélanyléthynyl)-benzoate de méthyle,
- acide 4-(4,4-diméthyl-thiochroman-8-ylsélanyléthynyl)-benzoïque,
- 4-(5,5,8,8-tétraméthyl-5,6,7,8-tétrahydro-naphtalèn-8-ylsélanyléthynyl)-benzoate de méthyle,
- acide 4-(5,5,8,8-tétraméthyl-5,6,7,8-tétrahydro-naphtalèn-8-ylsélanyléthynyl)-benzoïque,
- 4-[3-(1-adamantyl)-4-méthoxyphényl)-1-ylsélanyléthynyl]-benzoate de méthyle,
- acide 4-[3-(1-adamantyl)-4-méthoxyphényl)-1-ylsélanyléthynyl]-benzoïque,
- 4-[4-(1-adamantyl)-3-méthoxyphényl)-1-ylsélanyléthynyl]-benzoate de méthyle, et
- acide 4-[4-(1-adamantyl)-3-méthoxyphényl)-1-ylsélanyléthynyl]-benzoïque.

La présente invention a également pour objet les procédés de préparation des composés de formule (I) ci-dessus selon les schémas réactionnels donnés aux Tableaux A et B.

En se référant au Tableau A, les composés de formule (I), dans lesquels X représente le radical divalent 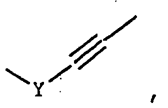 à savoir les composés de formule (Ia), peuvent être obtenus selon deux voies de synthèse différentes selon que Y = oxygène ou Y ≠ oxygène.

Lorsque X = oxygène, le produit de départ est le composé de formule (1) qui en présence d'une base telle que l'hydrure de potassium ou de sodium, est ensuite couplé avec du trichloroéthylène. Le produit dichloroéthylénique obtenu de formule (2) est ensuite soumis à l'action d'une base lithiée telle que le butyllithium dans un solvant tel que le THF pour conduire au composé acétylénique de formule (3). Ce dernier est alors couplé avec un halogénure d'aryle ou un halogénure d'hétéroaryle de préférence un dérivé iodé, en présence d'un catalyseur au palladium pour conduire aux composés de formule (IIa) avec Y = oxygène.

Lorsque Y ≠ oxygène, on prépare tout d'abord l'acétylure de lithium de formule (5) à partir du composé acétylénique aromatique ou hétéroaromatique (4), en présence d'un dérivé lithié tel que le butyllithium dans un solvant tel le THF. A partir de l'acétylure de lithium (5) qui n'est pas isolé, on réalise un couplage avec le composé de formule (6) dans un solvant tel que le THF et l'on obtient les composés de formule (Ia) avec Y ≠ oxygène.

A partir de ces derniers composés de formule (Ia) dans lesquels Y = S ou Se, il est possible d'accéder aux dérivés oxydés par oxydation à l'aide d'un agent oxydant tel que' l'acide métachloroperbenzoïque (AmCPB) ou le periodate de sodium.

En se référant maintenant au Tableau B, les composés de formule (I) dans lesquels X représente un radical divalent 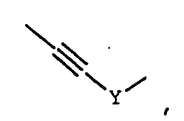 à savoir les composés de formule (Ic), peuvent être également obtenus selon deux voies de synthèse différentes selon que Y = oxygène ou Y ≠ oxygène.

Lorsque Y = oxygène, le produit de départ est un composé aromatique ou hétéroaromatique de formule (7), qui en présence d'une base telle que l'hydrure de potassium ou de sodium dans un solvant tel que le THF est ensuite couplé avec le trichloroéthylène. Le produit obtenu dichloroéthylénique (8) est ensuite soumis à l'action d'une base lithiée telle que le butyllithium dans le THF pour conduire au composé oxoacétylénique de formule (9). Ce dernier est alors couplé avec un halogénure d'aryle (10), de préférence un dérivé iodé, en présence d'un catalyseur au palladium pour conduire aux composés de formule (Ic) avec Y = oxygène.

Lorsque Y ≠ oxygène, le produit de départ est un composé acétylénique aromatique de formule (11) qui est transformé en dérivé lithié en présence de butyllithium par exemple dans un solvant tel que le THF. Le dérivé lithié acétylénique (12) qui n'est pas isolé, est alors couplé avec un composé aromatique ou hétéroaromatique de formule (13), la réaction de couplage étant effectuée dans un solvant tel que le THF. On obtient ainsi par cette voie de synthèse les composés de formule (Ic) avec Y ≠ oxygène.

A partir de ces derniers composés de formule (Ic) dans lesquels Y = S ou Se, il est également possible d'obtenir les dérivés oxydés par oxydation à l'aide d'un agent oxydant tel que l'acide métachloroperbenzoïque (AmCPB) ou le periodate de sodium.

Lorsque dans les composés selon l'invention le radical R₁ représente -COOH, ceux-ci sont préparés en protégeant la fonction acide carboxylique par un groupe protecteur du type alkyle.

Par saponification de la fonction ester en présence d'une base telle que l'hydroxyde de sodium ou de lithium dans un solvant alcoolique ou dans le THF, on obtient alors les acides libres correspondants.

Lorsque R₁ est -OH, les composés peuvent être obtenus à partir de l'acide correspondant par réduction en présence d'hydrure tel que l'hydrure de bore.

Lorsque R₁ est -CH=O, les composés peuvent être obtenus par oxydation des alcools correspondants à l'aide d'oxyde de manganèse ou de dichromate de pyridinium.

Lorsque R₁ est 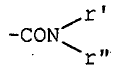 les composés peuvent être obtenus par transformation de l'acide correspondant en chlorure d'acide par exemple avec du chlorure de thionyle puis réaction avec l'ammoniaque ou une amine appropriée.

La présente invention a également pour objet à titre de médicament les composés de formule (I) telle que définie ci-dessus.

Les composés de formule générale (I) présentent une activité agoniste ou antagoniste vis-à-vis de l'expression d'un ou plusieurs marqueurs biologiques dans le test de différenciation des cellules (F9) de tératocarcinome embryonnaire de la souris (Skin Pharmacol. 3, p. 256-267, 1990) et/ou sur la différenciation des kératinocytes humains in vitro (Skin Pharmacol. 3 p. 70-85, 1990). Ces tests susmentionnés montrent les activités des composés dans les domaines de la différenciation et de la prolifération. Les activités peuvent aussi être mesurées dans des tests de transactivation cellulaire à l'aide de récepteurs recombinants RARs selon la méthode B.A. Bernard et al., Biochemical and Biophysical Research Communication, vol. 186, 977-983, 1992.

Les composés selon l'invention conviennent particulièrement bien dans les domaines des traitements suivants :
1) pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle,
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal),
3) pour traiter d'autres affections dermatologiques liées à un trouble de la kératinisation avec une composante inflammatoire et/ou immuno-allergique et notamment toutes les formes de psoriasis qu'il soit cutané, muqueux ou unguéal, et même le rhumatisme psoriatique, ou encore l'atopie cutanée telle que l'eczéma, ou l'atopie respiratoire ou encore l'hypertrophie gingivale ; les composés peuvent également être utilisés dans certaines affections inflammatoires ne présentant pas de trouble de la kératinisation,
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires,
5) pour traiter d'autres désordres dermatologiques tels que les dermatoses bulleuses et les maladies du collagène,
6) pour traiter certains troubles ophtalmologiques, notamment les cornéopathies,
7) pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique,
8) pour prévenir ou guérir les stigmates de l'atrophie épidermique et/ou dermique induite par les corticostéroïdes locaux ou systémiques, ou toute autre forme d'atrophie cutanée,
9) pour prévenir ou traiter les troubles de la cicatrisation ou pour prévenir ou pour réparer les vergetures,
10) pour lutter contre les troubles de la fonction sébacée tels que l'hyperséborrhée de l'acné ou la séborrhée simple,
11) pour traiter ou prévenir des états cancéreux ou précancéreux,
12) pour traiter les affections inflammatoires telles que l'arthrite,
13) pour traiter toute affection d'origine virale au niveau cutané ou général,
14) pour prévenir ou traiter l'alopécie,
15) pour traiter les affections dermatologiques ou générales à composante immunologique, et
16) pour traiter les affections du système cardiovasculaire telles que l'artériosclérose.

Dans les domaines thérapeutiques mentionnés ci-dessus, les composés selon l'invention peuvent être avantageusement employés en combinaison avec d'autres composés à activité de type rétinoïde, avec les vitamines D ou leurs dérivés, avec des corticostéroïdes, avec des anti-radicaux libres, des α-hydroxy ou α-cétoacides ou leurs dérivés ou bien encore avec des bloqueurs de canaux ioniques. Par vitamines D ou leurs dérivés, on entend par exemple les dérivés de la vitamine D₂ ou D₃ et en particulier la 1,25-dihydroxyvitamine D₃. Par anti-radicaux libres, on entend par exemple l'α-tocophérol, la Super Oxyde Dismutase ou SOD, l'Ubiquinol ou certains chélatants de métaux. Par α-hydroxy ou α-céto acides ou leurs dérivés, on entend par exemple l'acide lactique, l'acide malique, l'acide citrique, l'acide glycolique, l'acide mandélique, l'acide tartrique, l'acide glycérique ou l'acide ascorbique ou leurs sels, amides ou esters. Enfin, par bloqueurs de canaux ioniques, on entend par exemple le Minoxidil (2,4-diamino-6-pipéridino-pyrimidine-3-oxyde) et ses dérivés.

La présente invention a également pour objet des compositions pharmaceutiques contenant au moins un composé de formule (I) telle que définie ci-dessus, l'un de ses isomères optiques ou géométriques ou un de ses sels.

Les compositions pharmaceutiques sont destinées notamment au traitement des affections susmentionnées, et sont caractérisées par le fait qu'elles contiennent un support pharmaceutiquement acceptable et compatible avec le mode d'administration retenu, au moins un composé de formule (I), l'un de ses isomères optiques ou géométriques ou un de ses sels.

L'administration des composés selon l'invention peut être effectuée par voie entérale, parentérale, topique ou oculaire.

Par voie entérale, les compositions peuvent se présenter sous forme de comprimés, de gélules, de dragées, de sirops, de suspensions, de solutions, de poudres, de granulés, d'émulsions, de microsphères ou de nanosphères ou de vésicules lipidiques ou polymériques permettant une libération contrôlée. Par voie parentérale, les compositions peuvent se présenter sous forme de solutions ou de suspensions pour perfusion ou pour injection.

Les composés selon l'invention sont généralement administrés à une dose journalière d'environ 0,01 mg/kg à 100 mg/kg en poids corporel, et ceci à raison de 1 à 3 prises.

Par voie topique, les compositions pharmaceutiques à base des composés selon l'invention sont plus particulièrement destinées au traitement de la peau et des muqueuses et peuvent alors se présenter sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée du principe actif. Ces compositions par voie topique peuvent par ailleurs se présenter soit sous forme anhydre, soit sous une forme aqueuse, selon l'indication clinique.

Par voie oculaire, ce sont principalement des collyres.

Ces compositions à usage topique ou oculaire contiennent au moins un composé de formule (I) telle que définie ci-dessus, ou l'un de ses isomères optiques ou géométriques ou encore l'un de ses sels, à une concentration de préférence comprise entre 0,001 % et 5 % en poids par rapport au poids total de la composition.

Les composés de formule (I) selon l'invention trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les aspects néfastes du soleil ou dans le traitement des peaux physiologiquement sèches, pour prévenir et/ou pour lutter contre le vieillissement photoinduit ou chronologique.

Dans le domaine cosmétique, les composés selon l'invention peuvent par ailleurs être avantageusement employés en combinaison avec d'autres composés à activité de type rétinoïde, avec les vitamines D ou leurs dérivés, avec des corticostéroïdes, avec des anti-radicaux libres, des α-hydroxy ou α-céto acides ou leurs dérivés, ou bien encore avec des bloqueurs de canaux ioniques tous ces derniers composés étant tels que définis ci-dessus.

La présente invention a donc également pour objet une composition cosmétique qui est caractérisée par le fait qu'elle contient dans un support cosmétiquement acceptable, au moins un composé de formule (I) telle que définie ci-dessus ou l'un de ses isomères optiques ou géométriques ou l'un de ses sels, ladite composition cosmétique pouvant notamment se présenter sous forme d'une crème, d'un lait, d'une lotion, d'un gel, de microsphères ou de nanosphères ou de vésicules lipidiques ou polymériques, d'un savon ou d'un shampooing.

La concentration en composé de formule (I) dans les compositions cosmétiques selon l'invention est avantageusement comprise entre 0,001 % et 3 % en poids par rapport au'poids total de la composition.

Les compositions pharmaceutiques et cosmétiques selon l'invention peuvent en outre contenir des additifs inertes ou même pharmacodynamiquement ou cosmétiquement actifs ou des combinaisons de ces additifs et notamment : des agents mouillants, des agents dépigmentants tels que l'hydroquinone, l'acide azélalque, l'acide caféique ou l'acide kojique ; des émollients ; des agents hydratants comme le glycérol, le PEG 400, la thiamorpholinone et ses dérivés ou bien encore l'urée ; des agents antiséborrhéiques ou antiacnéiques, tels que la S-carboxyméthylcystéine, la S-benzylcystéamine, leurs sels ou leurs dérivés, ou le peroxyde de benzoyle ; des antibiotiques comme l'érythromycine et ses esters, la néomycine, la clindamysine et ses esters, les tétracyclines ; des agents antifongiques tels que le kétokonazole ou les polyméthylène-4,5 isothiazolidones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphénylimidazolidine 2,4-dione) ; des agents anti-inflammatoires non stéroïdiens ; des caroténoïdes et, notamment le β-carotène ; des agents anti-psoriatiques tels que l'anthraline et ses dérivés ; et enfin les acides eicosa-5,8,11,14-tétraynoïque et eicosa-5,8,11-trynoïque, leurs esters et amides.

Les compositions selon l'invention peuvent également contenir des agents d'amélioration de la saveur, des agents conservateurs tels que les esters de l'acide parahydroxybenzoïque, des agents stabilisants, des agents régulateurs d'humidité, des agents régulateurs de pH, des agents modificateurs de pression osmotique, des agents émulsionnants, des filtres UV-A et UV-B, des antioxydants tels que l'α-tocophérol, le butylhydroxyanisole ou le butylhydroxytoluène.

On va maintenant donner à titre d'illustration et sans aucun caractère limitatif, plusieurs exemples de préparation des composés actifs de formule (I) selon l'invention, ainsi que diverses formulations pharmaceutiques et cosmétiques à base de ces composés.

### EXEMPLES

### EXEMPLE 1 : 4-(5,5,8,8-tétraméthyl-5,6,7,8-tétrahydronaphtalèn-2-ylsulfanyléthynyl)benzoate de méthyle

### (a) 4-triméthylsilyléthynylbenzoate de méthyle

Dans on tricol, on introduit 21,5 g (0,1 mole) de 4-bromobenzoate de méthyle, 300 ml de triéthylamine et un mélange de 200 mg d'acétate de palladium et de 400 mg de triphénylphosphine. On ajoute ensuite 20 g (0,204 mole) de triméthylsilylacétylène, puis chauffe progressivement à 90°C durant 1 heure et laisse à cette température pendant 5 heures. On refroidit alors le milieu réactionnel, filtre le sel et évapore. On reprend le résidu avec 200 ml d'acide chlorhydrique (5 %) et 400 ml d'éther éthylique. On décante la phase éthérée, lave à l'eau, sèche sur sulfate de magnésium, et évapore. Le résidu obtenu est purifié par chromatographie sur colonne de silice, par élution avec du dichlorométhane. Après évaporation des solvants, on recueille 23 g (100 %) du dérivé attendu sous forme d'une huile incolore.

### (b) 4-éthynylbenzoate de méthyle

Dans un tricol, on introduit 38,33 g (226 mmoles) du produit précédemment obtenu dans 300 ml de méthanol. On ajoute ensuite 125 g de carbonate de potassium et le milieu est agité durant 48 heures à température ambiante. Le solvant est évaporé et le résidu obtenu est purifié par chromatographie sur colonne de silice, par élution avec du dichlorométhane. Après évaporation des solvants, on reprend le résidu dans l'heptane, et recueille, après filtration, 32 g (100 %) du dérivé attendu sous forme d'un solide jaune paille.

### (c) 4- (5,5,8,8-tétraméthyl-5,6,7,8-tétrahydronaphtalèn-2-ylsufanyléthynyl)benzoate de méthyle

Une solution de butyllithium 2,5 M dans l'hexane (20 mmoles, 8,1 ml) est ajoutée à une solution de 4-éthynylbenzoate de méthyle (3 g, 18,7 mmoles) dans le THF (300 ml) à -78°C. La température est maintenue 45 minutes, puis remontée à -40°C. On ajoute alors à cette température une solution de 5,6,7,8-tétrahydro-5,5,8,8-tétraméthylnaphtalèn-2-disulfure (J. Med. Chem. **1995,** *38,* 3171) (16,5 g, 37,4 mmoles) dans du THF (60 ml). Le mélange réactionnel est ensuite agité 1 heure à 0°C, puis versé sur un mélange d'éther éthylique et d'une solution saturée en chlorure d'ammonium. La phase organique est lavée 2 fois à l'eau, séchée sur sulfate de magnésium anhydre et concentrée à l'évaporateur rotatif sous vide à 40°C. Après chromatographie sur colonne de silice à l'aide d'un mélange d'heptane-chlorure de méthylène (60/40) on obtient, après évaporation, 1,9 g d'un solide blanc (27 %).
¹H (CDCl₃) : 1,28 (6H, s), 1,30 (6H, s), 1,69 (4H, s), 3,92 (3H, s), 7,25 à 7,31 (2H Ar, m), 7,42 (1H Ar, d, J=2Hz), 7,50 (1H Ar, d, J=7,5Hz), 8,00 (1H Ar, d, J=7,5Hz).
¹³C (CDCl₃) : 32,25 (CH₃), 34,60 (C), 35,02 (C), 35,36 (CH₂), 52,68 (OCH₃), 81,26 (C), 96,96 (C), 124,82 (CH Ar), 125,56 (CH Ar), 128,29 (C Ar), 128,37 (CH Ar), 128,85 (C Ar), 129,88 (C Ar), 130,04 (2 CH Ar), 131,29 (2 CH Ar), 144,66 (C Ar), 146,88 (C Ar), 166,95 (COO).

### EXEMPLE 2 : acide 4-(5,5,8,8-tétraméthyl-5,6,7,8-tétrahydro-naphtalèn-2-ylsulfanyléthynyl)benzoïque

Une solution de 4-(5,5,8,8-tétraméthyl-5,6,7,8-tétrahydro-naphtalèn-2-ylsulfanyléthynyl) benzoate de méthyle (590 mg, 1,6 mmoles) et d'hydroxyde de lithium (383 mg, 9,3 mmoles) dans du THF est chauffée à reflux 24 heures. Le mélange réactionnel est versé sur un mélange Et₂O/eau, acidifié à pH 1 par une solution d'acide chlorhydrique concentré, et extrait une fois à l'éther éthylique. Après décantation, la phase organique est lavée deux fois à l'eau, séchée sur sulfate de magnésium et concentrée à l'évaporateur rotatif sous vide à 40°C. Le solide obtenu est cristallisé dans l'heptane et l'on obtient 440 mg (77 %) d'un solide blanc. PF (point de fusion) = 193,5°C.
RMN δ ppm :
¹H (CDCl₃) : 1,28 (6H, s), 1,30 (6H, s), 1,69 (4H, s), 7,29 à 7,32 (2H Ar, m), 7,42 (1H Ar, d, J=2Hz), 7,53 (1H Ar, d, J=8,5Hz), 8,08 (1H Ar, d, J=8,5Hz).
¹³C (CDCl₃) : 31,44 (CH₃), 33,80 (C), 34,22 (C), 34,54 (CH₂), 81,30 (C), 100,01 (C), 124,06 (CH Ar), 124,81 (CH Ar), 127,59 (CH Ar), 127,94 (C Ar), 128,46 (C Ar), 129,87 (2 CH Ar), 130,49 (2 CH Ar), 143,93 (C Ar), 146,12 (C Ar), 171,06 (COO).

### EXEMPLE 3 : 4-(5,5,8,8-tétraméthyl-5,6,7,8-tétrahydronaphtalèn-2-ylsulfonyléthynyl)benzoate de méthyle

Une solution d'acide méta perbenzoïque (700 mg) dans du CHCl₃ (12 ml) est additionnée goutte à goutte, à 0°C sur une solution du produit de l'exemple 1 (500 mg, 1,3 mmoles) dans 6 ml de CHCl₃. Après 1 heure d'agitation, le mélange est concentré à l'évaporateur rotatif sous vide. Après chromatographie sur colonne de silice avec un mélange heptane-chlorure de méthylène (30/70) on obtient 280 mg d'un solide blanc (52 %). ¹H (CDCl₃) : 1,32 (6H, s), 1,34 (6H, s), 1,73 (4H, s), 3,93 (3H, s), 7,51 (2H Ar, d, J=8,3Hz), 7,60 (2H Ar; d, J=8,5Hz), 7,78 (1H Ar, dd, J1=8,5Hz, J2=2Hz), 7,98 à 8,05 (3H Ar, m). ¹³C (CDCl₃) : 31,63 (CH₃), 31,73 (CH₃), 34,58 (CH₂), 34,64 (CH₂), 34,85 (C), 34,98 (C), 52,52 (CH₃), 87,77 (C), 90,99 (C), 122,61 (C Ar), 124,35 (CH Ar), 125,99 (CH Ar), 128,04 (CH Ar), 129,68 (CH Ar), 132,44 (C Ar), 132,69 (CH Ar), 138,37 (C Ar), 146,89 (C Ar), 152,50 (C Ar), 165,83 (COO).

### EXEMPLE 4 : 4-(5,5,8,8-tétraméthyl-5,6,7,8-tétrahydro-naphtalèn-2-ylsélanyléthynyl)-benzoate de méthyle

### (a) 5, 6, 7,8-tétrahydro-5,5,8,8-tétraméthylnaphtalèn-2-disélénide

Une solution de tert-butyllithium 1,7 M dans du pentane (37,4 mmol, 22 ml) est additionnée à une solution de 2-bromo-5,6,7,8-tétrahydro-5,5,8,8-tétraméthylnaphtalène (4,22 g, 15,8 mmol) dans le THF (100 ml) à -78°C en 10 min. Le mélange est agité à 0°C 30 min. Le sélénium (1,33 g, 16,8 mmol) est additionné en 2 fois. Le mélange est agité à 0°C 15 min, puis à température ambiante 30 min. Une solution d'HCl 1N (40 ml) est additionnée, puis le mélange réactionnel est traité par de l'éther éthylique. La phase organique est lavée 2 fois à l'eau, séchée sur sulfate de magnésium anhydre et concentrée à l'évaporateur rotatif sous vide à 40°C. 10 ml d'éthanol et 50 mg de soude sont additionnés à l'huile obtenue. Le mélange est agité vigoureusement quelques minutes, puis est concentré à l'évaporateur rotatif sous vide à 40°C. Le solide obtenu est filtré sur silice (élution heptane) puis cristallisé dans un mélange éthanol / éther. On obtient après filtration 2,9 g (69 %) d'un solide orange.

RMN ¹H (CDCl₃) : 1,21 (6H, s), 1,25 (6H, s), 1,65 (4H, s), 7,20 (1H Ar, d, J=8,25 Hz), 7,38 (1H Ar, dd ,J=1,9 Hz, J=8,25 Hz), 7,51 (1H Ar, d, J=1,9 Hz).

### (b) 4- (5,5,8,8-tétraméthyl-5,6, 7,8-tétrahydro-naphtalèn-2-ylsélanyléthynyl)-benzoate de méthyle

Du brome (0,15 ml, 2,9 mmol), est additionné à une solution de 5,6,7,8-tétrahydro-5,5,8,8-tétraméthylnaphtalèn-2-disélénide (1,5 g, 2,8 mmol) dans du THF (3 ml). Le mélange est agité à température ambiante 2 h, puis le solvant est éliminé. De l'iodure de cuivre (2,15 g, 11,3 mmol), du 4-éthynyl-benzoate de méthyle (810 mg, 5 mmol) obtenu selon l'Exemple 1(b), et du DMF (15 ml), sont additionnés. Le mélange réactionnel est agité à température ambiante 3 h puis il est traité par de l'éther éthylique et une solution d'ammoniaque. La phase organique est lavée deux fois à l'eau, séchée sur sulfate de magnésium anhydre et concentrée à l'évaporateur rotatif sous vide à 40°C. Le résidu est recristallisé dans de l'heptane et l'on obtient après filtration 1,8 g (75 %) d'une poudre blanche. PF=90-1°C.
RMN ¹H (CDCl₃) : 1,28 (6H, s), 1,30 (6H, s), 1,69 (4H, s),
3,92 (3H, s), 7,29 (1H Ar, d, J=8,3 Hz), 7,36 (1H Ar, dd, J=1,9 Hz, J=8,3 Hz), 7,48 à 7,53 (3H Ar, m), 7,98 (2H Ar, d, J=8,5Hz).

### EXEMPLE 5 : Acide 4-(5,5,8,8-tétraméthyl-5,6,7,8-tétrahydronaphtalèn-2-ylsélanyléthynyl)-benzoïque

De l'hydroxyde de lithium (440 mg), est additionné à une solution de 4-(5,5,8,8-tétraméthyl-5,6,7,8-tétrahydro-naphtalèn-2-ylsélanyléthynyl)-benzoate de méthyle (740 mg, 1,74 mmol) obtenu à l'exemple 4, dans 15 ml de THF et 2 ml d'un mélange eau / méthanol (1/1). Le milieu réactionnel est chauffé 8 h à reflux. Il est ensuite versé sur un mélange éther éthylique / eau, acidifié à pH 1 par une solution d'acide chlorhydrique concentré et extrait à l'éther éthylique. Après décantation, la phase organique est lavée deux fois à l'eau, séchée sur sulfate de magnésium anhydre et concentrée à l'évaporateur rotatif sous vide à 40°C. Le résidu est recristallisé dans de l'heptane. Après filtration, on obtient 615 mg (86 %) d'une poudre blanche. PF=182°C.
RMN ¹H (CDCl₃) : 1,28 (6H, s), 1,30 (6H, s), 1,69 (4H, s), 7,29 (1H Ar, d, J=8,3 Hz), 7,36 (1H Ar, dd , J=1,9 Hz, J=8,3 Hz), 7,52 à 7,55 (3H Ar, m), 8,07 (2H Ar, d, J=8,5Hz).

### EXEMPLE 6 : 2-hydroxy-4-(5,5,8,8-tétraméthyl-5,6,7,8-tétrahydronaphtalèn-2-ylsélanyléthynyl)-benzoate de méthyle

### (a) 4-triméthylsilanyléthynyl-2-hydroxy-benzoate de méthyle

De manière analogue à l'exemple 1(a), à partir de 4,00 g (14,4 mmoles) de 4-iodo-2-hydroxy-benzoate de méthyle, on obtient 3,07 g (86%) du composé attendu, sous la forme d'une huile orangée.
RMN ¹H (CDCl₃) : 0,06 (s, 9H), 3,75 (s, 3H), 6,76 (dd, 1H, *J* = 8,2 / 1,5 Hz), 6,87 (d, 1H, *J* = 1,4 Hz), 7,56 (d, 1H, *J* = 8,2 Hz), 10,53 (s, 1H).

### (b) 4-éthynyl-2-hydroxy-benzoate de méthyle

On mélange dans un tricol de 500 ml, 3,07 g (12,4 mmol) de 4-triméthylsilanyléthynyl-2-hydroxy-benzoate de méthyle avec 50 ml de THF, et on ajoute, goutte à goutte, 13,7 ml d'une solution de fluorure de tétrabutylammonium (1 M / THF). Le milieu réactionnel est agité 1h à température ambiante puis est versé dans l'eau et extrait à l'éther éthylique. Après décantation, la phase organique est séchée sur sulfate de magnésium et concentrée.
On obtient 2,48 g (100%) d'une poudre beige. PF= 62°C.
RMN ¹H (CDCl₃) : 3,21 (s, 1H), 3,96 (s, 3H), 6,98 (dd, 1H, *J*= 8,2 / 1,5 Hz), 7,10 (d, 1H, *J* = 1,3 Hz), 7,78 (d, 1H, *J*= 8,2 Hz), 10,76 (s, 1H).

### (c) 2-hydroxy-4- (5,5,8,8-tétraméthyl-5,6,7,8-tétrahydro-naphtalèn-2-ylsélanyléthynyl)-benzoate de méthyle

De manière analogue à l'exemple 4(b), après réaction de 1,5 g (2,8 mmol) de 5,6,7,8-tétrahydro-5,5,8,8-tétraméthyl-naphtalèn-2-disélénide dans 2 ml de THF, avec du brome (0,15 ml, 2,9 mmol), on additionne de l'iodure de cuivre (2,15 g ; 11,3 mmol), du 4-éthynyl-2-hydroxy-benzoate de méthyle (890 mg ; 5 mmol) dans 10 ml de DMF. Après purification sur colonne de silice (dichlorométhane 10 - Heptane 90), on obtient 2,15 g (97%) du dérivé ester attendu sous forme de solide jaune. PF= 70°C.
RMN ¹H (CDCl3) : 1,28(d,12H) ; 1,69(s,4H) ; 3,95(s,3H) ; 6,94(dd,1H) ; 7,04(d,1H) ; 7,26 à 7,37(m,2H) ; 7,51(d,1H) ; 7,77(d,1H) ; 10,77(s,1H).
RMN ¹³C (CDCl3) : 31,8 ; 4*CH3/ 34,2 ; Cq/ 34,6 ; Cq/ 34,9 ; 2*CH2/ 52,4 ; CH3/ 75,1 ; Cq/ 101,6 ; Cq/ 111,9 ; Cq/ 119,7; CH/ 121,9 ; CH/ 124,5 ; Cq/ 127,0 ; CH/ 127, 8 ; CH/ 128,1 ; CH/ 129,9 ; CH/ 130,4 ; Cq/ 144,7 ; C/ 146,7 ; Cq/ 161,2 ; Cq/170,1 ; Cq.

### EXEMPLE 7 : Acide 2-hydroxy-4-(5,5,8,8-tétraméthyl-5,6,7,8-tétrahydro-naphtalèn-2-ylsélanyléthynyl)-benzoïque

Une solution de 2-hydroxy-4-(5,5,8,8-tétraméthyl-5,6,7,8-tétrahydro-naphtalèn-2-ylsélanyléthynyl)-benzoate de méthyle (1,2 g ; 2,72 mmol) obtenu à l'exemple 6(c) et de la soude (1,5 g ; 37,5 mmol) dans 20 ml de THF est chauffée 24h à reflux. Le mélange réactionnel est, ensuite, versé sur un mélange acétate d'éthyle / eau, acidifié à pH 1 par une solution d'acide chlorhydrique concentré, et extrait une fois à l'acétate d'éthyle. Après décantation, la phase organique est lavée deux fois à l'eau, séchée sur sulfate de magnésium et concentrée à l'évaporateur rotatif sous vide à 40°C. On obtient 1 g (86%) d'un solide jaune. PF=170°C.
RMN ¹H(DMSO) : 1,28(m,12H) ; 1,68(s,4H) ; 6,95(d,1H) ; 7,03(s,1H) ; 7,25 à 7,37(m,2H) ; 7,51(s,1H) ; 7.83(d,1H).
RMN ¹³C (DMSO) : 31,8 ; 4*CH3/ 34,2 ; Cq/ 34,6 ; Cq/ 34,9 ; 2*CH2/ 76,0 ; Cq/ 101,5 ; Cq/ 119,7 ; CH/ 122,1 ; CH/ 124,4 ; Cq/ 127,1 ; CH/ 127,9 ; CH/ 128,2 ; CH/ 130,9 ; CH/ 131,4 ; Cq/ 144.7 ; Cq/ 146,7 ; Cq/ 161,6 ; Cq/ 174,2 ; Cq.

### EXEMPLE 8 : 6-(4-méthoxyméthoxy-phényléthynylsélanyl)-1,1,4,4-tétraméthyl-1,2,3,4-tétrahydro-naphtalène

### (a) 1-iodo-4-méthoxyméthoxy-benzène

A une suspension d'hydrure de sodium 75% (872 mg ; 27,25 mmol) dans 20 ml de diméthylformamide, on ajoute 5 g (22,7 mmol) de 4-iodo-phénol. Le mélange est agité 30 minutes à température ambiante puis, 2,59 ml (34,1 mmol) de chlorure de méthoxyméthyle sont additionnés. La solution est agitée 2h puis le milieu est versé dans un mélange acétate d'éthyle / eau. Après décantation, la phase organique est lavée deux fois à l'eau, séchée sur sulfate de magnésium et concentrée à l'évaporateur rotatif sous vide à 40°C. On obtient 5,74 g (96 %) d'une huile incolore.
RMN ¹H(CDCl3) : 3,45(s,3H) ; 5,13(s,2H) ; 6,80(d, 2H) ; 7,55(d,2H)
RMN ¹³C (CDCl3) : 56,0 ; CH3/ 84,3 ; Cq/ 94,3 ; CH2/ 118,4 ; 2*CH/ 138,2 ; 2*CH/ 157,0 ; Cq

### (b) 1-triméthylsilyléthynyl-4-méthoxyméthoxy-benzène

Dans un tricol, on introduit 5,74 g (21,7 mmol) de 1-iodo-4-méthoxyméthoxy-benzène, 100 ml de triéthylamine et un mélange de 1,53 g (2,18 mmol) de dichloro-bis-(triphénylphosphine)-palladium et de 831 mg (4,37 mmol) d' iodure de cuivre. On ajoute, ensuite, 6,14 ml (43,5 mmol) de triméthylsilylacétylène et le milieu est agité 48h à température ambiante. Il est, alors, versé dans un mélange eau / acétate d'éthyle. La phase organique est lavée deux fois à l'eau et, après décantation, elle est séchée sur sulfate de magnésium et concentrée.

### (c) 1-éthynyl-4-méthoxyméthoxy-benzène

De manière analogue à l'exemple 1(b), par réaction du produit obtenu selon l'exemple 8(b) dans 50 ml de méthanol et avec du carbonate de potassium pendant 15h à température ambiante et après purification sur colonne de silice (dichlorométhane 20 - heptane 80), on obtient 840 mg (24%) du produit attendu sous forme d'une huile jaune.
RMN 1H (CDCl3) : 3,00(s,1H) ; 3,46(s,3H) ; 5,17(s,2H) ; 6,97(d,2H) ; 7,42(d,2H).
RMN ¹³C (CDCl3) : 56,1 ; CH3/ 76,1 ; Cq/ 83,5 ; CH/ 94,2 ; CH2/ 115,4 ; Cq/ 116,1 ; CH/ 133,6 ; CH/ 157,6 ; Cq.

### (d) 6-(4-méthoxyméthoxy-phényléthynylsélanyl)-1,1,4,4-tétraméthyl-1,2,3,4-tétrahydro-naphtalène

De manière analogue à l'exemple 4(b), après réaction de 1,3 g (2,44 mmol) de 5,6,7,8-tétrahydro-5,5,8,8-tétraméthylnaphtalèn-2-disélénide dans 2 ml de THF, avec du brome (0,13 ml, 2,5 mmol), on additionne de l'iodure de cuivre (1,86 g ; 9,8 mmol), du 1-éthynyl-4-méthoxyméthoxy-benzène (713 mg ; 4,4 mmol) dans 10 ml de DMF. Après purification sur colonne de silice (dichlorométhane 20 - Heptane 80), on obtient 1,7 g (90%) du dérivé attendu sous forme d'une huile jaune.
RMN ¹H (CDCl₃) : 1,27(m,12H) ; 1,67(s,4H) ; 3,47(s,3H) ; 5,18(s,2H) ; 6,98(dd,2H) ; 7,01 à 7,51(m,5H).
RMN ¹³C (CDCl3) : 31,8 ; 4*CH3/ 34,1 ; Cq/ 34,5 ; Cq/ 34,9 ; 2*CH2/ 56,1 ; CH3/ 68,3 ; Cq/ 77,5 ; Cq/ 102,0 ; Cq/ 116,1 ; 2*Ch/ 116,7 ; Cq/ 125,3 ; Cq/ 133,3 ; 2*CH/ 144,2 ; Cq/146,5 ; Cq/ 157,4 ; Cq.

### EXEMPLE 9 : Acide 6-(5,5,8,8-tétraméthyl-5,6,7,8-tétrahydronaphtalèn-2-ylsélanyléthynyl)-nicotinique

### (a) 6-triméthylsilyléthynyl-pyridine-3-carboxylate d'éthyle

De manière analogue à l'exemple 1(a), à partir de 4 g (14,4 mmol) de 6-iodo-pyridine-3-carboxylate de méthyle, on obtient 3,29 g (92%) du composé attendu, sous la forme d'une poudre beige. PF= 55°C.
RMN ¹H (CDCl₃) δ 0,10 (s, 9H), 1,22 (t, 2H, *J* = 7,1 Hz), 4,23 (q, 3H, *J* = 7,1 Hz), 7,33 (d, 1H, *J* = 8,2 Hz), 8,06 (dd, 1H, *J* = 8,1 / 2,1 Hz), 8,97 (d, 1H, *J* = 2,1 Hz).

### (b) 6-éthynyl-nicotinate d'éthyle

De manière analogue à l'exemple 6(b), à partir de 3,29 g (13,3 mmoles) de 6-triméthylsilyléthynyl-nicotinate d'éthyle, on obtient 1,00 g (43%) du composé attendu, sous formé de paillettes beiges. PF=35°C.
RMN ¹H (CDCl₃) δ 1,42 (t, 3H, *J* = 7,1 Hz), 3,33 (s, 1H), 4,42 (q, 2H, *J*= 7,2 Hz), 7,56 (d, 1H, *J* = 8,1 Hz), 8,28 (dd, 1H, *J* = 8,1 / 2,1 Hz), 9,18 (d, 1H, *J* = 2,0 Hz).

### (c) 6-(5,5,8,8-tétraméthyl-5,6,7,8-tétrahydro-naphtalèn-2-ylsélanyléthynyl)-nicotinate d'éthyle

De manière analogue à l'exemple 4(b), après réaction de 1,84 g (3,4 mmol) de 5,6,7,8-tétrahydro-5,5,8,8-tétraméthylnaphtalèn-2-disélénide dans 2 ml de THF, avec du brome (0,18 ml, 3,49 mmol), on additionne de l'iodure de cuivre (2,64 g ; 13,9 mmol), du 6-éthynyl-nicotinate d'éthyle (1g ; 5,7 mmol) dans 10 ml de DMF. On obtient 1,95 g (78%) du dérivé attendu sous forme d'une huile brune.
RMN ¹H (CDCl3) : 1,28 à 1,30(m,12H) ; 1,40(t,3H) ; 1,69(s,4H); 4,41(q,2H) ; 7,12 à 7,59(m,4H) ; 8,24(dd,1H) ; 9,16(d,1H).

### (d) Acide 6-(5,5,8,8-tétraméthyl-5,6,7,8-tétrahydro-naphtalèn-2-ylsélanyléthynyl)-nicotinique

De manière analogue à l'exemple 7, par réaction de 600 mg (1,36 mmol) de 6-(5,5,8,8-tétraméthyl-5,6,7,8-tétrahydro-naphtalèn-2-ylsélanyléthynyl)-nicotinate d'éthyle dans 30 ml de THF et avec 1 g de soude, on obtient, après trituration dans l'heptane, 200 mg (36%) du composé attendu sous forme d'un solide jaune. PF=128°C.
RMN ¹H (CDCl3) : 1,27 à 1,30(m,12H) ; 1,68(s,4H) ; 7,26 à 7,52(m,5H) ; 8,32(d,1H) ; 9,26(s,1H)
RMN ¹³C (CDCl3) : 31,8 ; 4*CH3/ 34,2 ; Cq/ 34,6 ; Cq/ 34,8 ; CH2/ 34,9 ; CH2/ 78,6 ; Cq/ 101,4 ; Cq/ 123,6 ; Cq/ 123,8 ; Cq/ 125,8 ; CH/ 127,8 ; CH/ 128,4 ; CH/ 128,5 ; CH/ 137,9 ; CH/ 145,1 ; Cq/ 146,8 ; Cq/ 147,0 ; Cq/ 151,5 ; CH/ 169,0 ; Cq.

### EXEMPLE 10 : N-(4-hydroxy-phényl)-4-(5,5,8,8-tétraméthyl-5,6,7,8-tétrahydro-naphtalèn-2-ylsélanyléthynyl)-benzamide

Une solution de 250 mg (0,63 mmol) de l'acide 4-(5,5,8,8-tétraméthyl-5,6,7,8-tétrahydro-naphtalèn-2-ylsélanyléthy-nyl)-benzoïque obtenu à l'exemple 5, de 169 mg (1,25 mmol) de 1-hydroxybenzotriazole, de 240 mg (1,25 mmol) de 1-(3-diméthylaminopropyl)-3-éthylcarbodiimide (EDC) et de 82 mg (0,75 mmol) de 4-aminophénol dans 20 ml de THF, est agitée à température ambiante pendant 15h. On ajoute, alors, de l'eau et de l'acétate d'éthyle. Après agitation et décantation, la phase aqueuse est extraite avec de l'acétate d'éthyle. Les phases organiques sont, alors, réunies et lavées avec de l'eau et séchées sur sulfate de magnésium et concentrées à l'évaporateur rotatif sous vide à 40°C. Le produit est purifié sur colonne de silice (acétate d'éthyle 20 - heptane 80). On obtient 200 mg (65 %) d'un solide blanc. PF=202°C.
RMN ¹H (DMSO) : 1,23 (s, 6H) ; 1,25 (s, 6H) ; 1,64 (s, 4H); 6,72 à 6,76 (d, 2H) ; 7,39 (c, 1H) ; 7,51 à 7,55 (d, 2H) ; 7,59 à 7,61 (d, 2H) ; 7,64 à 7,67 (d, 2H) ; 7,95 à 7,98 (d, 2H) ; 9,28 (s, 1H) ; 10,10 (s, 1H).

### EXEMPLE 11 : 5-(5,5,8,8-tétraméthyl-5,6,7,8-tétrahydro-naphtalèn-2-ylsélanyléthynyl)-pyridine-2-carboxylate de méthyle

### (a) 5-triméthylsilyléthynyl-pyridine-2-carboxylate de méthyle

De manière analogue à l'exemple 1(a), à partir de 7 g (26,6 mmol) de 5-iodo-pyridine-2-carboxylate de méthyle, on obtient 4,25 g (68%) du composé attendu sous forme d'une poudre orangée. PF= 45°C.
RMN ¹H (CDCl₃) δ 0,28 (s, 9H), 4,01 (s, 3H), 7,87 (dd, 1H, *J*= 8,1 / 2,0 Hz), 8,08 (d, 1H, *J* = 8,1 Hz), 8,77 (d, 1H, *J* = 1,3 Hz).

### (b) 5-éthynyl-pyridine-2-carboxylate de méthyle

De manière analogue à l'exemple 6(b), à partir de 2,25 g (9,6 mmol) de 5-triméthylsilyléthynyl-pyridine-2-carboxylate de méthyle, on obtient 380 mg (24%) du composé attendu sous forme d'une poudre jaune. PF= 40-5°C.
RMN 1H(CDCl₃) δ 3,40 (s, 1H), 4,02 (s, 3H), 7,93 (dd, 1H, *J*= 8,1 / 2,0 Hz), 8,12 (d, 1H, *J* = 8,1 Hz), 8,83 (d, 1H, *J* = 1,9 Hz).

### (c) 5- (5,5,8,8-tétraméthyl-5,6,7,8-tétrahydro-naphtalèn-2-ylsélanyléthynyl)-pyridine-2-carboxylate de méthyle

De manière analogue à l'exemple 4(b), après réaction de 918 mg (1,73 mmol) de 5,6,7,8-tétrahydro-5,5,8,8-tétraméthylnaphtalèn-2-disélénide dans 2 ml de THF, avec du brome (0,092 ml, 1,78 mmol), on additionne de l'iodure de cuivre (1,62 g ; 8,5 mmol), du 5-éthynyl-pyridine-2-carboxylate de méthyle (500 mg ; 3,1 mmol) dans 10 ml de DMF. Après trituration dans l'heptane, on obtient 420 mg (32%) du dérivé attendu sous forme d'un solide jaune. PF= 75°C.
RMN ¹H (CDCl3) : 1,28 à 1,29(d,12H) ; 1,69(s,4H) ; 4,02(s,3H); 7,27 à 7,37(m,2H) ; 7,54(d,1H) ; 7,84(dd,1H) ; 8,11(d,1H) ; 8,77(s,1H).
RMN ¹³C (CDCl3) : 31,7 ; 4*CH3/ 34,2 ; Cq/ 34,6 ; Cq/ 34,8 ; 2*CH2/ 53,0 ; CH3/ 79,2 ; Cq/ 98,3 ; Cq/ 123,9 ; 2*Cq/ 124,5 ; CH/ 127,4 ; CH/ 128,2 ; CH/ 128,3 ; CH/ 138,7 ; CH/ 145,1 ; Cq/ 145,8 ; Cq/ 146,9 ; Cq/ 151,6 ; CH/ 165,2 ; Cq.

### EXEMPLE 12 : 2-(4-chloro-phénylsélanyléthynyl)-5,5,8,8-tétraméthyl-5,6,7,8-tétrahydro-naphtalène

De manière analogue à l'exemple 4(b), après réaction de 2 g (5,25 mmol) de bis-(4-chlorophényl)-disélénide dans 5 ml de THF, avec du brome (0,266 ml, 5,15 mmol), on additionne de l'iodure de cuivre (4,11 g ; 21,6 mmol), du 6-éthynyl-1,1,4,4-tétraméthyl-1,2,3,4-tétrahydro-naphtalène (2,18 g (10 mmol) (décrit dans la demande EP 0 661 258 A1) dans 20 ml de DMF, et après purification sur colonne de silice (heptane), on obtient 1,85 g (45%) du dérivé attendu sous forme d'une huile incolore.
RMN ¹H (CDCl3) : 1.28(s,12H) ; 1.68(s,4H) ; 7.26 à 7.30(m,4H) ; 7.46 à 7.52(m,3H).

### EXEMPLE 13 : 4-(3,5,5,8,8-pentaméthyl-5,6,7,8-tétrahydro-naphtalèn-2-ylsélanyléthynyl)-benzoate de méthyle

### (a) 5,6,7,8-tétrahydro-3,5,5,8,8-pentaméthylnaphtalène-2-disélénide

De manière analogue à l'exemple 4(a), par réaction de 4,4 g (15,8 mmol) de 2-bromo-(5,6,7,8-tétrahydro-3,5,5,8,8-pentaméthylnaphtalène), avec 22 ml de tert-butyllithium et du sélénium (1,33 g, 16,8 mmol) dans 100 ml de THF, on obtient 3,26 g (74%) du dérivé sélénié attendu sous forme d'un solide jaune. (PF=126°C).
RMN ¹H (CDCl₃) : 1,14 (6H, s), 1,23 (6H, s), 1,61 (4H, s), 2,35 (3H, s), 7,05 (1H Ar, s), 7,55 (1H Ar, s).

### (b) 4-(3,5,5,8,8-pentaméthyl-5,6,7,8-tétrahydro-naphtalèn-2-ylsélanyléthynyl)-benzoate de méthyle

De manière analogue à l'exemple 4(b), après réaction de 1,5 g (2,75 mmol) de 5,6,7,8-tétrahydro-3,5,5,8,8-pentaméthylnaphtalèn-2-disélénide dans 5 ml de THF, avec du brome (0,15 ml, 2,9 mmol), on additionne de l'iodure de cuivre (2,1 g ; 11,05 mmol), du 4-éthynylbenzoate de méthyle (790 mg ; 4,94 mmol) dans 20 ml de DMF, et après trituration dans l'heptane, on obtient 1,57 g (70%) du dérivé attendu sous forme d'un solide blanc. PF=104°C.
RMN ¹H (CDCl3) : 1,27 à 1,29(m,12H) ; 1,68(s,4H) ; 2,36(s,3H); 3,91(s,3H) ; 7,12(s,1H) ; 7,50(d,2H) ; 7,73(s,1H) ; 8,00(d,2H).
RMN ¹³C (CDCl3) : 21,4 ; CH3/ 32,3 ; 2*CH3/ 32,4 ; 2*CH3/34,5 ; Cq/ 34,8 ; Cq/ 35,5 ; 2*CH2/ 52,7 ; CH3/ 75,0 ; Cq/102,2 ; Cq/ 125,9 ; Cq/ 128,5 ; Cq/ 129,1 ; 2*CH/ 129,8 ; Cq/130,1 ; 2*CH/ 131,5 ; 2*CH/ 134,9 ; Cq/ 144,7 ; Cq/ 145,3 ; Cq/ 167,0 ; Cq.

### EXEMPLE 14 : Acide 4-(3,5,5,8,8-pentaméthyl-5,6,7,8-tétrahydro-naphtalèn-2-ylsélanyléthynyl)-benzoïque

De manière analogue à l'exemple 7, par réaction de 1,35 g (3,07 mmol) de 4-(3,5,5,8,8-pentaméthyl-5,6,7,8-tétrahydro-naphtalèn-2-ylsélanyléthynyl)-benzoate de méthyle dans 20 ml de THF et avec 3 g de soude, on obtient, après trituration dans l'heptane, 1,05 g (80%) du composé attendu sous forme d'un solide blanc. PF=240°C.
RMN ¹H (CDCl3) : 1,27 à 1,30(m,12H) ; 1,68(s,4H) ; 2,35(s,3H); 7,13(s,1H) ; 7,50(d,2H) ; 7,71(s,1H) ; 8,00(d,2H).
RMN ¹³C (CDCl3) : 20,5 ; CH3/ 31,5 ; 4*CH3/ 33,6 ; Cq/ 33,9 ; Cq / 34,6 ; 2*CH2/ 73,6 ; Cq/ 101,6 ; Cq/ 125,0 ; Cq/ 127,1 ; Cq/ 127,9 ; CH/ 128,3 ; CH/ 129,4 ; 2*CH/ 130,5 ; 2*CH/133,8 ; Cq/ 143,9 ; 2*Cq/ 144,5 ; Cq/ 167,5 ; Cq.

### EXEMPLE 15 : 2-hydroxy-4-(3,5,5,8,8-pentaméthyl-5,6,7,8-tétrahydronaphtalèn-2-ylsélanyléthynyl)-benzoate de méthyle

De manière analogue à l'exemple 4(b), après réaction de 1 g (1,78 mmol) de 5,6,7,8-tétrahydro-3,5,5,8,8-pentaméthylnaphtalèn-2-disélénide dans 5 ml de THF, avec du brome (0,092 ml, 1,78 mmol), on additionne de l'iodure de cuivre (1,36 g ; 7,15 mmol), du 4-éthynyl-2-hydroxy-benzoate de méthyle (566 mg ; 3,2 mmol) obtenu selon l'exemple 6(b) dans 10 ml de DMF, et après trituration dans l'heptane, on obtient 715 mg (49%) du dérivé attendu sous forme d'un solide marron. PF=102°C.
RMN ¹H (CDCl3) : 1,20(s,6H) ; 1,23(s,6H) ; 1,60(s,4H) ; 2,28(s,3H) ; 3,87(s,3H) ; 6,87(dd,1H) ; 6,97(d,1H) ; 7,04(s,1H) ; 7,64(s,1H) ; 7,71(d,1H) ; 10,70(s,1H).
RMN ¹³C (CDCl3) :20,7 ; CH3/ 31,7 ; 4*CH3/ 33,8 ; Cq/ 34,1 ; Cq/ 34,8 ; 2*CH2/ 74,9 ; Cq/ 101,4 ; Cq/ 111,7 ; Cq/ 119,4 ; CH/ 121,6 ; CH/ 125,1 ; Cq/ 128,4 ; 2*CH/ 129,7 ; CH/ 130,3 ; Cq/ 134,2 ; Cq/ 144,1 ; Cq/ 144,7 ; Cq/ 161,1 ; Cq/ 169,9 ; Cq.

### EXEMPLE 16 : Acide 2-hydroxy-4-(3,5,5,8,8-pentaméthyl-5,6,7,8-tétrahydronaphtalèn-2-ylsélanyléthynyl)-benzoïque

De manière analogue à l'exemple 7, par réaction de 500 mg (1,1 mmol) de 2-hydroxy-4-(3,5,5,8,8-pentaméthyl-5,6,7,8-tétrahydronaphtalèn-2-ylsélanyléthynyl)-benzoate de méthyle dans 20 ml de THF et avec 500 mg de soude, on obtient 464 mg (99%) du composé attendu sous forme d'un solide marron. PF= 248°C.
RMN ¹H (CDCl3+DMSO) : 0,89(s,6H) ; 0,92(s,6H) ; 1,30(s,4H); 1,96(s,3H) ; 6,55(dd,1H) ; 6,60(s,1H) ; 7,31(s,1H) ; 7,43(d,1H) ; 10,96(sb,1H).
RMN ¹³C (CDCl3+DMSO) : 20,6 ; CH3/ 31,6 ; 4*CH3/ 34,0 ; Cq/34,6 ; Cq/ 34,7 ; 2*CH2/ 74,1 ; Cq/ 101,6 ; Cq/ 112,5 ; Cq/118,9 ; CH/ 121,3 ; CH/ 125,0 ; Cq/ 128,0 ; CH/ 128,3 ; CH/129,6 ; Cq/ 130,4 ; CH/ 133,9 ; Cq/ 144,0 ; Cq/ 144,5 ; Cq/161,4 ; Cq/ 171,9 ; Cq.

### EXEMPLE 17 : 6-(3,5,5,8,8-pentaméthyl-5,6,7,8-tétrahydro-naphtalèn-2-ylsélanyléthynyl)-nicotinate d'éthyle

De manière analogue à l'exemple 4(b), après réaction de 1 g (1,78 mmol) de 5,6,7,8-tétrahydro-3,5,5,8,8-pentaméthylnaphtalèn-2-disélénide dans 5 ml de THF, avec du brome (0,092 ml, 1,78 mmol), on additionne de l'iodure de cuivre (1,36 g ; 7,15 mmol), du 6-éthynyl-nicotinate d'éthyle (463 mg ; 2,64 mmol) dans 10 ml de DMF, on obtient 1,06 g (88%) du dérivé attendu sous forme d'un solide marron. PF=95°C.
RMN ¹H (CDCl3) : 1,20(s,6H) ; 1,24(s,6H) ; 1,34(t,3H) ; 1,61(s,3H) ; 4,33(q,2H) ; 7,07(s,1H) ; 7,38(d,1H) ; 7,67(s,1H); 8,17(dd,1H) ; 9,08(d,1H).
RMN ¹³C (CDCl3) : 13,9 ; CH3/ 20,9 ; CH3/ 31,5 ; 4*CH3/ 33,7 ; Cq/ 34,0 ; Cq/ 34,6 ; 2*CH2/ 61,2 ; CH2/ 77,2 ; Cq/ 101,2 ; Cq/ 124,2 ; Cq/ 124,3 ; Cq/ 125,2 ; CH/ 128,4 ; CH/ 129,4 ; CH/ 134,8 ; Cq/ 136,8 ; Cq/ 144,0 ; Cq/ 145,1 ; Cq/ 146,3 ; Cq/ 150,8 ; CH/ 164,5 ; Cq.

### EXEMPLE 18 : Acide 6-(3,5,5,8,8-pentaméthyl-5,6,7,8-tétrahydro-naphtalèn-2-ylsélanyléthynyl)-nicotinique

De manière analogue à l'exemple 7, par réaction de 800 mg (1,73 mmol) de 6-(3,5,5,8,8-pentaméthyl-5,6,7,8-tétrahydro-naphtalèn-2-ylsélanyléthynyl)-nicotinate d'éthyle dans 20 ml de THF et avec 800 mg de soude, on obtient, après purification sur colonne de silice (acétate d'éthyle), 135 mg (19%) du composé attendu sous forme d'un solide jaune. PF=185°C.
RMN ¹H (CDCl3) : 1,27(s,6H) ; 1,31(s,6H) ; 1,68(s,4H) ; 2,40(s,3H) ; 7,15(s,1H) ; 7,26(s,1H) ; 7,49(d,1H) ; 7,74(s,1H) ; 8,32(d,1H) ; 9,25(s,1H).
RMN ¹³C (CDCl3) : 21,7 ; CH3/ 32,2 ; 4*CH3/ 34,5 ; Cq/ 34,7 ; Cq/ 35,3 ; 2*CH2/ 78,9 ; Cq/ 101,7 ; Cq/ 124,0 ; Cq/ 124,9 ; Cq/ 126,1 ; CH/ 129,1 ; CH/ 130,2 ; CH/ 135,6 ; Cq/ 1382 ; CH/144,8 ; Cq/ 145,9 ; Cq/ 147,6 ; Cq/ 152,0 ; CH/ 169,5 ; Cq.

### EXEMPLE 19 : N-(4-hydroxy-phényl)-6-(3,5,5,8,8-pentaméthyl-5,6,7,8-tétrahydro-naphtalèn-2-ylsélanyléthynyl)-nicotinamide

De manière analogue à l'exemple 10, par réaction de 300 mg (0,72 mmol) de l'acide 6-(3,5,5,8,8-pentaméthyl-5,6,7,8-tétrahydro-naphtalèn-2-ylsélanyléthynyl)-nicotinique avec 194 mg (1,45 mmol) de 1-hydroxybenzotriazole, 300 mg (1,45 mmol) de 1,3-dicyclohexylcarbodiimide et 95 mg (0,87 mmol) de 4-aminophénol dans 20 ml de THF. Après purification sur colonne de silice (acétate d'éthyle 20 - heptane 80), on obtient 20 mg (6%) d'un solide jaune. PF= 172°C.
RMN ¹H (DMSO) : 1,17 à 1,19(m,12H) ; 1,56(s,4H) ; 2,27(s,3H) ; 6,68(d,2H) ; 7,21(s,1H) ; 7,46(d,2H) ; 7,58(d,1H) ; 7,64(s,1H); 8,22(dd,1H) ; 8,99(s,1H)9,30(s,1H) ; 10,2(s,1H).
RMN ¹³C (DMSO) : 31,6 ; 4*CH3/ 33,5 ; CH2/ 33,8 ; CH2/ 34,0 ; Cq/ 34,5,Cq/ 47,6 ; CH3/ 74,9 ; Cq/ 102,0 ; Cq/ 115,2 ; 2*CH/122,3 ; 2*CH/ 124,4 ; Cq/ 125,9 ; CH/ 128,7 ; CH/ 128,9 ; CH/130,4 ; Cq/ 134,8 ; Cq/ 136,1 ; CH/ 144,0 ; Cq/ 144,1 ; Cq/145,1 ; Cq/ 149,3 ; Cq/ 154,1 ; Cq/ 156,8 ; Cq.

### EXEMPLE 20 : N-butyl-6-(3,5,5,8,8-pentaméthyl-5,6,7,8-tétrahydro-naphtalèn-2-ylsélanyléthynyl)-nicotinamide

De manière analogue à l'exemple 10, on fait réagir 300 mg (0,72 mmol) de l'acide 6-(3,5,5,8,8-pentaméthyl-5,6,7,8-tétrahydro-naphtalèn-2-ylsélanyléthynyl)-nicotinique avec 194 mg (1,45 mmol) de 1-hydroxybenzotriazole, 300 mg (1,45 mmol) de 1,3-dicyclohexylcarbodiimide et 63,5 mg (0,87 mmol) de butylamine dans 20 ml de THF. Après purification sur colonne de silice (acétate d'éthyle 20 - heptane 80), on obtient 60 mg (17%) d'un solide jaune. PF= 172°C.
RMN ¹H (CDCl3) : 0,97(t,3H) ; 1,27 à 1,37(m,12H) ; 1,37 à 1,46(m,4H) ; 1,68(s,4H) ; 2,39(s,3H) ; 3,47(q,2H) ; 6,13(m,1H) ; 7,14(s,1H) ; 7,46(d,1H) ; 7,74; (s,1H) ; 8,07(dd,1H) ; 8,87(s,1H).
RMN ¹³C (CDCl3) : 13,8 ; CH3/ 20,2 ; CH2/ 21,2 ; CH3/ 31,7 ; 4*CH3/ 34,0 ; Cq/ 34,3 ; Cq/ 35,0 ; 2*CH2/ 40,0 ; CH2/ 76,2 ; Cq/ 101,2 ; Cq/ 124,7 ; Cq/ 126,0 ; CH/ 128,7 ; CH/ 129,7 ; CH/ ; CH/ 35,1 ; Cq/ 135,3 ; CH/ 144,3 ; Cq/ 145,4 ; Cq/145,5 ; Cq/ ; Cq.

### EXEMPLE 21 : Morpholin-4-yl-[6-(3,5,5,8,8-pentaméthyl-5,6,7,8-tétrahydro-naphtalèn-2-ylsélanyléthynyl)-pyridin-3-yl]-méthanone

De manière analogue à l'exemple 10, on fait réagir 300 mg (0,72 mmol) de l'acide 6-(3,5,5,8,8-pentaméthyl-5,6,7,8-tétrahydro-naphtalèn-2-ylsélanyléthynyl)-nicotinique avec 194 mg (1,45 mmol) de 1-hydroxybenzotriazole, 300 mg (1,45 mmol) de 1,3-dicyclohexylcarbodiimide et 75,7 mg (0,87 mmol) de morpholine dans 20 ml de THF. Après purification sur colonne de silice (acétate d'éthyle 20 - heptane 80), on obtient 60 mg (17%) d'une huile incolore.
RMN ¹H (CDCl3) : 1,27 à 1,32(m,12H) ; 1,68(s,4H) ; 2,39(s,3H); 3,81(sbr,8H) ; 7,13(s,1H) ; 7,45(d,1H) ; 7,71 à 7,75(m,2H) ; 8,61(d,1H).
RMN ¹³C (CDCl3) : 21,2 ; CH3/ 31,8 ; 4*CH3/ 34,1 ; Cq/ 34,3 ; Cq/ 35,0 ; 2*CH2/ 66,8 ; 4*CH2/ 75,5 ; Cq/ 101,1 ; Cq/ 124,7 ; Cq/ 126,0 ; CH/ 128,7 ; CH/ 129,4 ; Cq/ 129,7 ; CH/ 135,1 ; Cq/ 135,5 ; CH/ 144,3 ; Cq/ 144,5 ; Cq/ 145,4 ; Cq/ 148,3 ; CH/ 167, 4 ; Cq

### EXEMPLE 22 : 5-(3,5,5,8,8-pentaméthyl-5,6,7,8-tétrahydro-naphtalèn-2-ylsélanyléthynyl)-pyridine-2-carboxylate de méthyle

De manière analogue à l'exemple 4(b), après réaction de 945 mg (1,68 mmol) de 5,6,7,8-tétrahydro-3,5,5,8,8-pentaméthylnaphtalèn-2-disélénide dans 5 ml de THF, avec du brome (0,092 ml, 1,78 mmol), on additionne de l'iodure de cuivre (1,32 g ; 6,95 mmol), du 5-éthynyl-pyridine-2-carboxylate de méthyle (500 mg; 3,1 mmol) dans 10 ml de DMF, après trituration dans l'heptane, on obtient 1 g (73%) du dérivé attendu sous forme d'un solide jaune. PF= 52°C.
RMN ¹H (CDCl3) : 1,27 à 1,29(m,12H) ; 1,68(s,4H) ; 2,37(s,3H); 4,02(s,3H) ; 7,14(s,1H) ; 7,71(s,1H) ; 7,85(dd,1H) ; 8,02(s,1H) ; 8,11(d,1H).
RMN ¹³C (CDCl3) : 20,7 ; CH3/ 31,5 ; 2*CH3/ 31,6 ; 2*CH3/33,7 ; Cq/ 34,0 ; Cq/ 34,6 ; 2*CH2/ 52,7 ; CH3/ 78,9 ; Cq/98,1 ; Cq/ 123,7 ; Cq/ 124,2 ; CH/ 124,5 ; Cq/ 128,4 ; CH/128,5 ; CH/ 134,3 ; Cq/ 138,3 ; CH/ 144,0 ; Cq/ 144,9 ; Cq/145,5 ; Cq/ 151,2 ; CH/ 162,2 ; Cq.

### EXEMPLE 23 : Acide 5-(3,5,5,8,8-pentaméthyl-5,6,7,8-tétrahydro-naphtalèn-2-ylsélanyléthynyl)-pyridine-2-carboxylique

De manière analogue à l'exemple 7, par réaction dé 800 mg (1,73 mmol) de 5-(3,5,5,8,8-pentaméthyl-5,6,7,8-tétrahydro-naphtalèn-2-ylsélanyléthynyl)-pyridine-2-carboxyiate de méthyle dans 20 ml de THF et avec 2 g de soude, on obtient, après trituration dans l'heptane, 580 mg (75%) du composé attendu sous forme d'un solide blanc. PF=164°C.
RMN ¹H (CDCl3) : 1,28(s,6H) ; 1,30(s,6H) ; 1,69(s,4H) ; 2,39(s,3H) ; 7,16(s,1H) ; 7,69(s,1H) ; 7,93(d,1H) ; 8,17(dbr,1H) ; 8,66(sbr,1H).
RMN ¹³C (CDCl3) : 21,2 ; CH3/ 31,8 ; 2*CH3/ 3,9 ; 2*CH3/34,1 ; Cq/ 34,3 ; Cq/ 34,9 ; 2*CH2/ 124,6 ; Cq/ 128,8 ; CH/129,3 ; CH/ 134,9 ; Cq/ 139,8 ; CH/ 144,4 ; 2*Cq/ 145,5 ; 2*Cq.

### EXEMPLE 24 : [4-(5,5,8,8-tétraméthyl-5,6,7,8-tétrahydro-naphtalèn-2-ylsélanyléthynyl)-phényl]-méthanol

Une solution 1M d'hydrure de diisobutylaluminium dans le toluène (4 ml, 4 mmol) est additionnée à 0°C, goutte à goutte à une solution de 4-(5,5,8,8-tétraméthyl -5,6,7,8-tétrahydro-naphtalèn-2-ylsélanyléthynyl)-benzoate de méthyle obtenu selon l'exemple 4 (750 mg, 1,8 mmol) dans du toluène (20 ml). La solution est agitée 4h à 0°C, puis traité par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C. On obtient 418 mg (60 %) d'une huile incolore.
RMN ¹H (CDCl₃) : 1,26 (s, 6H), 1,28 (s, 6H), 1,76 (s, 4H), 4,67 (s, 2H), 7,24 à 7,37 (m, 4H), 7,46 (d, 2H, *J*= 8,2 Hz), 7,52 (d, 1H, *J* = 1,9 Hz).

### EXEMPLE 25 : 4-(5,5,8,8-tétraméthyl-5,6,7,8-tétrahydro-naphtalèn-2-yléthynylsulfanyl)-benzoate de méthyle

De manière analogue à l'exemple 1(c), par réaction de 234 mg (1,1 mmol) de 6-éthynyl-1,1,4,4-tétraméthyl-1,2,3,4-tétrahydronaphtalène dans 5 ml de THF, avec du butyllithium 2,5 M (0,4 ml, 1 mmol) et du 2,2'-dithiobis(benzoate de méthyle) (267 mg ; 0,8 mmol), on obtient, après purification sur colonne de silice (dichloromethane 30 - heptane 70), le dérivé attendu sous forme d'un solide blanc.
RMN ¹H (CDCl₃) : 1,28 (6H, s), 1,29 (6H, s), 1,69 (4H, s), 3,91 (3H, s), 7,30 (2H Ar, s), 7,49 à 7,54 (3H Ar, m), 8,0 (2H Ar, d, J=6,9Hz).

### EXEMPLE 26 : 4-(3,5,5,8,8-pentaméthyl-5,6,7,8-tétrahydro-naphtalèn-2-ylsélanyléthynyl)-phénol

### (a) Acétate de 4-triméthylsilyléthynyl-phényle

De manière analogue à l'exemple 1(a), à partir de 4,63 g (17,7 mmol) d'acétate de 4-iodophényle, on obtient 3,72 g (90%) du composé attendu sous forme d'une poudre jaune. PF= 45°C.
RMN¹H/CDCl3 : 0,05(s ; 9H) ; 2,10(s,3H) ; 6,84(dt,2H) ; 7,28(dt,2H).
RMN ¹³C/CDCl3 : 0,00 ; 2*CH3/ 21,2 ; CH3/ 94,4 ; Cq/ 104,3 ; Cq/ 120,9 ; Cq/ 121,2 ; 2*CH/ 133,2 ; 2*CH/ 150,7 ; Cq/169,1 ; Cq.

### (b) Acétate de 4-(5,5,8,8-tétraméthyl-5,6,7,8-tétrahydro-naphtalèn-2-ylsélanyléthynyl)-phényle

De manière analogue à l'exemple 4(b), après réaction de 1,39 g (2,4 mmol) de 5,5,8,8-tétraméthyl-5,6,7,8-tétrahydro-naphtalèn-2-disélénide dans le THF, avec du brome (0,22 ml, 4,3 mmol), on additionne de l'iodure de cuivre (1,82 g, 9,6 mmol) et de l'acétate de 4-trimethyllsilylethynyl-phényl ester (1 g ; 4,3 mmol) dans le DMF à 80°C pendant 15h, après purification sur colonne de silice (dichlorométhane 20 - heptane 80), on obtient 220 mg (16%) du dérivé attendu sous forme d'une huile jaune.
RMN¹H/CDCl3 : 1,19(d,12H) ; 1,59(s,4H) ; 2,22(s,3H) ; 2,26(s,3H) ; 6,97 à 7,02(m,3H) ; 7,39 à 7,42(dd,2H) ; 7,65(s,1H).
RMN 13C/CDCl3 : 19,2 ; CH3/ 19,5 ; CH3/ 30,3 ; 4*CH3/ 32,4 ; Cq/ 32,7 ; Cq/ 33,4 ; CH2/ 33,5 ; CH2/ 68,7 ; Cq/ 99,9 ; Cq/119,5 ; Cq/ 120,1 ; 2*CH/ 124,2 ; Cq/ 126,7 ; CH/ 126,9 ; CH/131,0 ; 2*CH/ 133,0 ; Cq/ 142,5 ; Cq/ 143,5 ; Cq/ 138,0 ; Cq/167,5 ; Cq.

### (c) 4-(3,5,5,8,8-pentaméthyl-5,6,7,8-tétrahydro-naphtalèn-2-ylsélanyléthynyl)-phénol

Un mélange d'acétate de 4-(5,5,8,8-tétraméthyl-5,6,7,8-tétrahydro-naphtalèn-2-ylsélanyléthynyl)-phényle (500 mg, 1,1 mmol) de carbonate de potassium (160 mg, 1,1 mmol) dans le méthanol (20 ml) est agité 24 h à température ambiante puis est traité par de l'éther éthylique et de l'eau. La phase organique est lavée deux fois à l'eau, séchée sur sulfate de magnésium anhydre et concentrée à l'évaporateur rotatif sous vide à 40°C. Le produit est purifié sur colonne de silice (Acétate d'éthyle 20 - heptane 80). On obtient 300 mg (66%) d'une huile claire.
RMN¹H/CDCl3 : 1,25 à 11,27(m,12H) ; 1,66(s,4H) ; 2,35(s,3H) ; 6,77(d,2H) ; 7,09(s,1H) ; 7,38(dd,2H) ; 7,73(s,1H).
RMN 13C/CDCl3 : 20,3 ; CH3/ 31,4 ; 4*CH3/ 33,6 ; 2*Cq/ 34,6 ; 2*CH2/ 67,2 ; Cq/ 103,7 ; Cq/ 115,3 ; Cq/ 115,6 ; 2*CH/127,7 ; Cq/ 128,5 ; 2*CH/ 133,0 ; 2*CH/ 133,6 ; Cq/ 143,6 ; Cq/ 143,9 ; Cq/ 156,0 ; Cq.

### EXEMPLE 27 : 4-(4-hydroxy-5,5,8,8-tétraméthyl-5,6,7,8-tétrahydro-naphtalèn-2-ylsélanyléthynyl)-benzoate d'éthyle

De manière analogue à l'exemple 4(b), après réaction de 1 g (1,5 mmol) de 4-méthoxyméthoxy-5,5,8,8-tétraméthyl-5,6,7,8-tétrahydro-naphtalèn-2-disélénide dans le THF, avec du brome (0,092 ml, 1,78 mmol), on additionne de l'iodure de cuivre et du 4-trimethyllsilyléthynylbenzoate d'éthyle (644 mg ; 2,8 mmol) dans le DMF à 80°C pendant 15h. Après purification sur colonne de silice (dichlorométhane 20 - heptane 80), on obtient 220 mg (16%) du dérivé attendu sous forme d'une huile jaune.
RMN¹H/CDCl3 : 1,29(s,6H) ; 1,37 à 1,43(m,9H) ; 1,65(q,4H) ; 4,39(q,2H) ; 5,72(s,1H) ; 7,26(s,1H) ; 7,43(s,1H) ; 7,55(d,2H) ; 8,03(d,2H).

### EXEMPLE 28 : 4-(4-méthoxyméthoxy-5,5,8,8-tétraméthyl-5,6,7,8-tétrahydro-naphtalèn-2-ylsélanyléthynyl)-benzoate d'éthyle

De manière analogue à l'exemple 4(b), après réaction de 1 g (1,5 mmol) de 4-méthoxyméthoxy-5,5,8,8-tétraméthyl-5,6,7,8-tétrahydro-naphtalèn-2-disélénide dans le THF, avec du brome (0,092 ml, 1,78 mmol) on additionne de l'iodure de cuivre et du 4-triméthyllsilyléthynylbenzoate d'éthyle (644 mg ; 2,8 mmol) dans le DMF à 80°C pendant 15h. Après purification sur colonne de silice (dichlorométhane 20 - heptane 80), on obtient 420 mg (31%) du dérivé attendu sous forme d'une huile jaune.
RMN¹H/CDCl3 : 1,17(q,6H) ; 1,31(m,9H) ; 1,49 à 1,57(m,4H) ; 3,38(s,3H) ; 4,25(q,2H) ; 5,10(s,2H) ; 7,08(d,1H) ; 7,14(d,1H) ; 7,41(d,2H) ; 7,88(d,2H).

### EXEMPLE 29 : Acide 4-(4-méthoxyméthoxy-5,5,8,8-tétraméthyl-5,6,7,8-tétrahydro-naphtalèn-2-ylsélanyléthynyl)-benzoïque

De manière analogue à l'exemple 7, par réaction de 300 mg de 4-(4-méthoxyméthoxy-5,5,8,8-tétraméthyl-5,6,7,8-tétrahydronaphtalèn-2-ylsélanyléthynyl)-benzoate d'éthyle ester dans 30 ml de THF et avec 500 mg de soude, on obtient, après trituration dans l'heptane, le composé attendu sous forme d'un solide blanc.
RMN¹H/CDCl3 : 1,28 (s,6H) ; 1,39 (s,6H) ; 1,66 (m, 2H) ; 3,51 (s, 3H) ; 5,23 (s, 2H) ; 7,19 (d, 1H, J=1,8 Hz) ; 7,25 (d, 1H, J=1,8 Hz) ; 7,56 (d,2H, J=8,5 Hz) ; 8,06 (d,2H, J=8,5Hz).

### EXEMPLE 30 : [4-(5,5,8,8-tétraméthyl-5,6,7,8-tétrahydro-naphtalèn-2-ylsélanyléthynyl)-phényl]-carbaldéhyde

Un mélange de [4-(5,5,8,8-tétraméthyl-5,6,7,8-tétrahydro-naphtalèn-2-ylsélanyl éthynyl)-phényl]-méthanol obtenu à l'exemple 24 (280 mg, 0,7 mmol) et de dichromate de pyridinium (526 mg, 1,4 mmol) dans le dichlorométhane (10ml) est agité à température ambiante 4h. Après filtration sur silice et concentration à l'évaporateur rotatif sous vide à 40°C, on obtient 173 mg (63%) du produit attendu sous forme d'une huile jaune.
RMN ¹H (CDCl₃) : 1,28 (s, 6H), 1,30 (s, 6H), 1,70 (s, 4H), 4,67 (s, 2H), 7,23 (1H Ar , d, J=8,3Hz), 7,29 (1H Ar, dd, J=1,9 Hz, J = 8,3 Hz), 7,52 à 7, 59 (3H Ar, *m*), 7,84 (1H Ar, d, J=6,7 Hz), 9,99 (.H, s).

### EXEMPLE 31 : 4-(4,4-diméthyl-thiochroman-8-ylsélanyléthynyl)-benzoate de méthyle

### (a) 2-brome-1-(3-méthylbut-2-ènylthio)benzène

Dans un tricol, on introduit 19,30 g (102,0 mmoles) de 2-bromothiophénol, 160 ml de DMF et 15,50 g (112,0 mmoles) de carbonate de potassium. On ajoute goutte à goutte 13 ml (112,0 mmoles) de 1-bromo-3-méthyl-2-butène et agite à la température ambiante pendant deux heures. On verse le milieu réactionnel dans l'eau, extrait avec de l'acétate d'éthyle, décante la phase organique, lave à l'eau, sèche sur sulfate de magnésium, évapore. On recueille 26,00 g (99%) du composé attendu, sous la forme d'une huile orangée.
¹H NMR (CDCl3) d 1,65 (s, 3H), 1,73 (s, 3H), 3,56 (d, 2H, J = 7,7 Hz), 5,32 (td, 1H, J = 7,7 / 1,4 Hz), 6,96 à 7,06 (m, 1H), 7,22 à 7,26 (m, 2H), 7,52 (d, 1H, J = 7,7 Hz).

### (b) 4, 4-diméthyl-8-bromothiochromane

Dans un tricol, on introduit 26,00 g (102,0 mmoles) de 2-bromo-1-(3-méthylbut-2-ènylthio)benzène, 180 ml de toluène et 23,20 g (122,0 mmoles) d'acide para-toluène sulfonique. On chauffe à reflux pendant quatre heures et évapore le milieu réactionnel à sec. On reprend par une solution aqueuse d'hydrogénocarbonate de sodium, extrait avec de l'acétate d'éthyle, décante la phase organique, sèche sur sulfate de magnésium, évapore. Le résidu obtenu est purifié par chromatographie sur colonne de silice, élué avec de l'heptane. On recueille 20,00 g (76%) du composé attendu, sous la forme d'une huile orangée.
¹H NMR (CDCl3) d 1,33 (s, 6H), 1,94 (t, 2H, J = 6,0 Hz), 3,04 (t, 2H, J = 6,1 Hz), 6,89 (t, 1H, J = 7,9 Hz), 7,34 (d, 2H, J = 7,9 Hz).

### (c) 4, 4-diméthyl-thiochroman-8-disélénide

Un cristal d'iode, du magnésium (208 mg, 8,56 mmol) et quelques gouttes d'une solution de 4,4-diméthyl-8-bromothiochromane (2g, 7,78 mmol) dans l'éther éthylique (15ml) sont chauffés jusqu'à l'amorçage de l'organomagnésien. Le reste de la solution est alors additionnée goutte à goutte. Le milieu réactionnel est chauffé 2h, puis le sélénium (615 mg, 7,78 mmol) est additionné à température ambiante. L'agitation est poursuivie 30 mn puis, une solution d'HCl 1N est additionnée. Le mélange réactionnel est traité par de l'éther éthylique. La phase organique est lavée 2 fois à l'eau, séchée sur sulfate de magnésium anhydre et concentrée à l'évaporateur rotatif sous vide à 40°C. De l'éthanol et de l'hydroxyde de sodium sont additionnés à l'huile obtenue. Le mélange est agité vigoureusement quelques minutes, puis est concentré à l'évaporateur rotatif sous vide à 40°C.
Le produit est purifié sur colonne de silice (dichlorométhane 20 - heptane 80).
On obtient 300 mg (15%) d'un solide blanc.
RMN ¹H (CDCl₃) : 1,33 (6H, s), 1,96 (2H, m), 3,09 (2H, m), 6,93 (1H Ar, t, J=7,8 Hz), 7,26 (1H Ar, dd, J=7,8 Hz, J=1,3 Hz), 7,47 (1H Ar, dd, J=7,8 Hz, J=1,3 Hz).

### (d) 4-(4,4-diméthyl-thiochroman-8-ylsélanyléthynyl)-benzoate de méthyle

De manière analogue à l'exemple 4(b), après réaction de 300 mg (1,9 mmol) de 4,4-diméthyl-thiochroman-8-disélénide dans 2 ml de THF, avec du brome (0,117 ml, 2,2 mmol), on; additionne de l'iodure de cuivre (780 mg), du 4-éthynyl-benzoate de méthyle (562 mg ; 3,5 mmol) dans 20 ml de DMF, et après purification sur colonne de silice (dichlorométhane 20 - héptane 80), on obtient le dérivé attendu sous forme d'un solide jaune.
RMN ¹H (CDCl₃) : 1,35 (6H, s), 1,97 (2H, m), 3,10 (2H, m), 3,93 (3H, s), 7,07 (1H Ar, t, J=7,8 Hz), 7,31 (1H Ar, dd, J=7,8 Hz, J=1,3 Hz), 7,55 (2H Ar, d, J=8,5 Hz), 7,59 (1H Ar, dd, J=7,8 Hz, J=1,3 Hz), 8, 00 (2H Ar, d, J=8,5 Hz).

### EXEMPLE 32 : Acide 4-(5,5,8,8-tétraméthyl-5,6,7,8-tétrahydro-naphtalèn-2-yléthynylsulfanyl)-benzoïque

De manière analogue à l'exemple 2, par réaction du 4-(5,5,8,8-tétraméthyl-5,6,7,8-tétrahydro-naphtalèn-2-yléthynylsulfanyl)-benzoate de méthyle dans le THF, on obtient, après cristallisation dans l'heptane, le dérivé attendu sous forme d'un solide blanc.
RMN ¹H (CDCl₃) : 1,28 (6H, s), 1,29 (6H, s), 1,70 (4H, s), 7,30 (2H Ar, s), 7,43 à 7,50 (3H Ar, t), 7,99 (2H Ar, d, J=7,5Hz).

### EXEMPLES DE FORMULATION

### EXEMPLE 1 :

On décrit ci-après diverses formulations pharmaceutiques et cosmétiques à base des composés selon l'invention.

### A - VOIE ORALE

### (a) Comprimé de 0,2 g

- Composé de l'exemple 1 10,001 g
- Amidon 0,114 g
- Phosphate bicalcique 0,020 g
- Silice 0,020 g
- Lactose 0,030 g
- Talc 0,010 g
- Stéarate de magnésium 0,005 g

Dans cet exemple, le composé de l'exemple 1 peut être remplacé par la même quantité d'un composé des exemples 4, 6, 11, 13 ou 15.

### (b) Suspension buvable en ampoules de 5 ml

- Composé de l'exemple 3 20,001 g
- Glycérine 0,500 g
- Sorbitol à 70 % 0,500 g
- Saccharinate de sodium 0,010 g
- p-hydroxybenzoate de méthyle 0,040 g
- Arôme qs
- Eau purifiée q.s.p. 5 ml

### (c) Comprimé de 0,8 g

- Composé de l'exemple 2 0,500 g
- Amidon prégélatinisé 0,100 g
- Cellulose microcristalline 0,115 g
- Lactose 0,075 g
- Stéarate de magnésium 0,010 g

Dans cet exemple, le composé de l'exemple 2 peut être remplacé par la même quantité d'un composé des exemples 6, 11, 14 ou 28.

### (d) Suspension buvable en ampoules de 10 ml

- Composé de l'exemple 3 0,200 g
- Glycérine 1,000 g
- Sorbitol à 70 % 1,000 g
- Saccharinate de sodium 0,010 g
- p-hydroxybenzoate de méthyle 0,080 g
- Arôme qs
- Eau purifiée q.s.p. 10 ml

### B - VOIE TOPIQUE

### (a) Onguent

- Composé de l'exemple 2 20,020 g
- Myristate d'isopropyle 81,700 g
- Huile de vaseline fluide 9,100 g
- Silice ("Aérosil 200" vendue par Degussa) 9,180 g

### (b) Onguent

- Composé de l'exemple 1 0,300 g
- Vaseline blanche codex 100 g

### (c) Crème eau-dans-l'huile non ionique

- Composé de l'exemple 1 0,100 g
- Mélange d'alcools de lanoline émulsifs, de cires et d'huiles ("Eucérine anhydre" vendu par BDF) 39,900 g
- p-hydroxybenzoate de méthyle 0,075 g
- p-hydroxybenzoate de propyle 0,075 g
- Eau déminéralisée stérile q.s.p. 100 g

Dans cet exemple, le composé de l'exemple 1 peut être remplacé par la même quantité d'un composé des exemples 4, 16, 22, 27 ou 32.

### (d) Lotion

- Composé de l'exemple 3 0,100 g
- Polyéthylène glycol (PEG 400) 69,900 g
- Ethanol à 95 % 30,000 g

### (e) Onguent hydrophobe

- Composé de l'exemple 1 0,300 g
- Myristate d'isopropyle 36,400 g
- Huile de silicone ("Rhodorsil 47V300" vendu par Rhône-Poulenc) 36,400 g
- Cire d'abeille 13,600 g
- Huile de silicone ("Abil 300.000 cst" vendu par Goldschmidt) 100 g

### (f) Crème huile-dans-l'eau non ionique

- Composé de l'exemple 2 1,000 g
- Alcool cétylique 4,000 g
- Monostéarate de glycérol 2,500 g
- Stéarate de PEG 502,500 g
- Beurre de karité 9,200 g
- Propylène glyco 12,000 g
- p-hydroxybenzoate de méthyle 0,075 g
- p-hydroxybenzoate de propyle 0,075 g
- Eau déminéralisée stérile 100 g

Dans cet exemple, le composé de l'exemple 2 peut être remplacé par la même quantité d'un composé des exemples 5, 9, 12, 19, 24 et 32.

### TEST D'ACTIVITE

Résultats de tests de différentiation des cellules (F9) de tératocarcinome embryonnaire de souris pour identifier des molécules agonistes RARs tels que décrits dans Skin Pharmacol. 3, p.256-267, 1990.

Après traitement par les composés des exemples cités dans le tableau suivant, les cellules F9 de tératocarcinome embryonaire de souris se différencient en cellules endodermiques. Cette différenciation est caractérisée par la sécrétion dans le milieu de culture de l'activateur du plasminogène.

L'activité du produit est exprimée par la valeur d'AC₅₀ représentant la concentration du produit testé qui produit la moitié.de la quantité maximale d'activateur de plasminogène sécrété.

| Exemples | F9 AC₅₀ (nM) |
|---|---|
| Composé 1 | 20 |
| Composé 2 | 1 |
| Composé 4 | 4 |
| Composé 5 | 21 |
| Composé 16 | 33 |
| Composé 18 | 34 |

Ces résultats indiquent que les composés des exemples 1, 2, 4, 5, 16 et 18 sont des composés agonistes RARs.

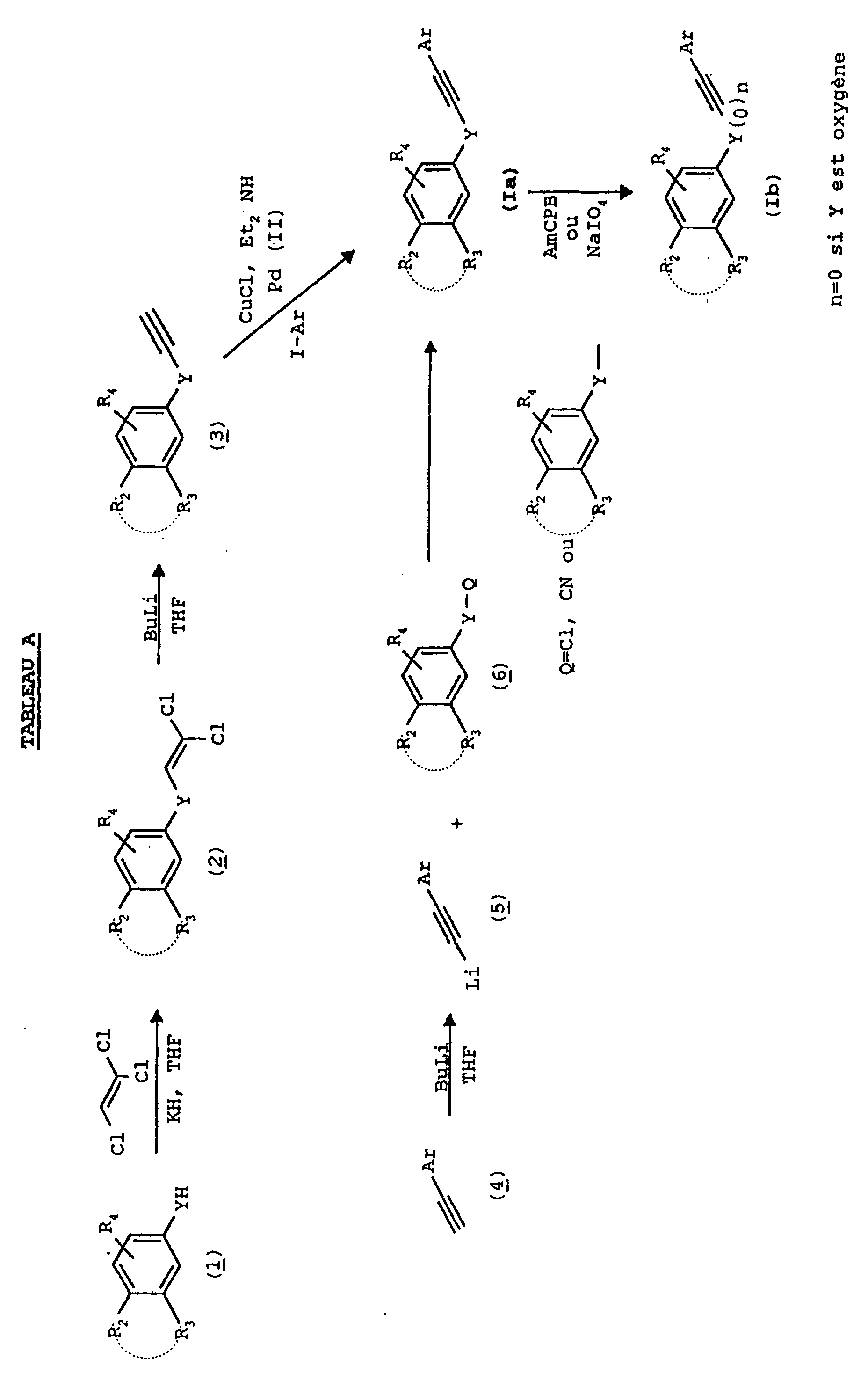

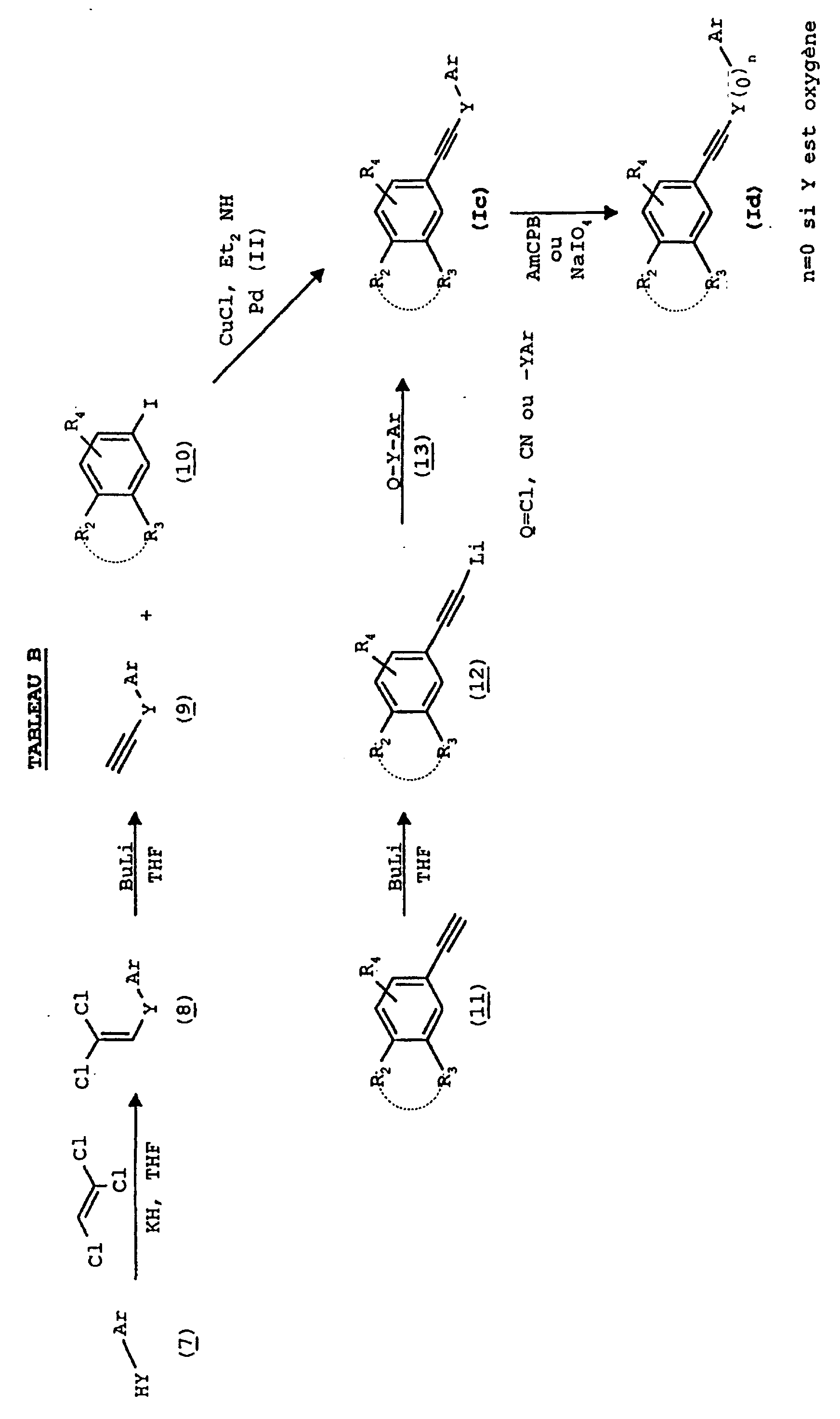

## Revendications

1. Composés bi-aromatiques reliés par une liaison hétéroéthynylène, **caractérisés en ce qu'**ils répondent à la formule générale (I) suivante : 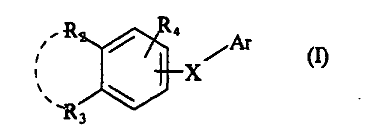 dans laquelle :
Ar représente un radical choisi par les formules (a) et (b) suivantes : 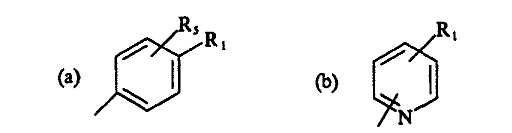
R₁ représente un atome d'halogène, -CH₃, -CH₂-OR₇, -OR₇ ou -COR₈,
R₂ et R₃, identiques ou différents, représentent H, alkyle, linéaire ou ramifié, en C₁-C₂₀, cycloalkyle en C₃-C₁₂, ou -OR₇, l'un au moins de R₂ et R₃ étant alkyle, linéaire ou ramifié, en C₁-C₂₀, ou cycloalkyle en C₃-C₁₀, ou
R₂ et R₃, pris ensemble forment un cycle à 5 ou 6 chaînons, pouvant comporter au moins un méthyle et/ou pouvant être interrompu par un hétéroatome choisi parmi O ou S,
R₄ et R₅ représentent H, un atome d'halogène, alkyle, linéaire ou ramifié, en C₁-C₂₀, -OR₇, ou un radical polyéther,
R₇ représente H, alkyle, linéaire ou ramifié en C₁-C₁₀ ou -COR₉,
R₈ représente H, alkyle, linéaire ou ramifié en C₁-C₁₀, -OR₁₀ ou 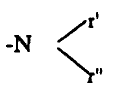
R₉ représente alkyle, linéaire ou ramifié en C₁-C₁₀,
R₁₀ représente H, alkyle, linéaire ou ramifié en C₁-C₂₀, mono- ou polyhydroxyalkyle, allyle, aryle ou aralkyle,
r' et r", identiques ou différents, représentent H, alkyle en C₁-C₁₀, mono- ou polyhydroxyalkyle, aryle ou pris ensemble avec l'atome d'azote, forment un hétérocycle,
X représente un radical divalent qui de droite à gauche ou inversement a pour formule : 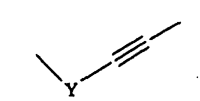
dans laquelle :
Y représente S(O)ₙ ou Se(O)_{n'},
n et n' étant 0, 1 ou 2,
sous réserve que lorsque Ar est un radical de formule (a) ci-dessus dans laquelle R₁=-CH₃, un atome d'halogène ou un radical -OR₇ et R₅=H, alors l'un des radicaux R₂ ou R₃ est différent de -CH₃,
et les sels des composés de formule (I) lorsque R₁ représente une fonction acide carboxylique ainsi que les isomères optiques desdits composés de formule (I).

2. Composés selon la revendication 1, **caractérisés en ce qu'**ils se présentent sous forme d'un sel d'un métal alcalin ou alcalino-terreux, ou encore de zinc ou d'une amine organique.

3. Composés selon l'une des revendications 1 ou 2, **caractérisés en ce que** le radical alkyle en C₁-C₁₀ est choisi dans le groupe constitué par les radicaux méthyle, éthyle, isopropyle, butyle, tertiobutyle, hexyle, 2-éthyl-hexyle ou octyle.

4. Composés selon l'une quelconque des revendications précédentes, **caractérisés en ce que** le radical alkyle, linéaire ou ramifié, en C₁-C₂₀, est choisi dans le groupe constitué par les radicaux méthyle, éthyle, propyle, 2-éthyl-hexyle, octyle, dodécyle, hexadécyle et octadécyle.

5. Composés selon l'une quelconque des revendications précédentes, **caractérisés en ce que** le radical cycloalkyle en C₃-C₁₂ est choisi dans le groupe constitué par les radicaux cyclopropyle, cyclopentyle, cyclohexyle, 1-méthylcyclohexyle ou 1-adamantyle.

6. Composés selon l'une quelconque des revendications précédentes, **caractérisés en ce que** le radical polyéther est choisi dans le groupe constitué par les radicaux méthoxyméthoxy, méthoxyéthoxy et méthoxyéthoxyméthoxy.

7. Composés selon l'une quelconque des revendications précédentes, **caractérisés en ce que** le radical monohydroxyalkyle est choisi dans le groupe constitué par les radicaux 2-hydroxyéthyle, 2-hydroxypropyle ou 3-hydroxypropyle.

8. Composés selon l'une quelconque des revendications précédentes, **caractérisés en ce que** le radical polyhydroxyalkyle est choisi dans le groupe constitué par les radicaux 2, 3-dihydroxypropyle, 2, 3, 4-trihydroxybutyle, 2, 3, 4, 5-tétrahydroxypentyle ou le reste du pentaérythritol.

9. Composés selon l'une quelconque des revendications précédentes, **caractérisés en ce que** le radical aryle est un radical phényle.

10. Composés selon l'une quelconque des revendications précédentes, **caractérisés en ce que** le radical aralkyle est choisi dans le groupe constitué par les radicaux benzyle ou phénéthyle.

11. Composés selon l'une quelconque des revendications précédentes, **caractérisés en ce que** le radical hétérocyclique est choisi dans le groupe constitué par les radicaux pipéridino, morpholino, pyrrolidino, pipérazino, ou pipérazino substitué en position 4 par un alkyle en C₁-C₆ ou un mono- ou polyhydroxyalkyle.

12. Composés selon l'une quelconque des revendications précédentes, **caractérisés en ce que** l'atome d'halogène est choisi dans le groupe constitué par le fluor, le chlore et le brome.

13. Composés selon l'une quelconque des revendications précédentes, **caractérisés par le fait qu'**ils répondent à la formule générale suivante : 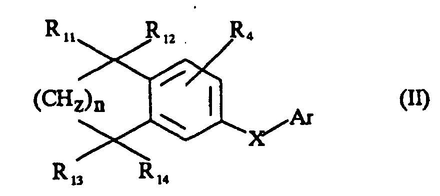 dans laquelle :
Ar représente un radical de formule: 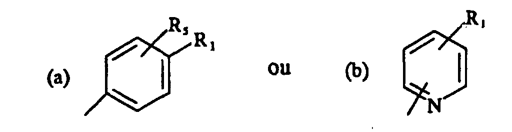
R₁, R₄, R₅ et X étant tels que définis à la revendication 1,
R₁₁, R₁₂, R₁₃ et R₁₄, identiques ou différents représentent H ou -CH₃, et
n est 1 ou 2.

14. Composés selon l'une quelconque des revendications 1 à 12, **caractérisés en ce qu'**ils répondent à la formule générale suivante : 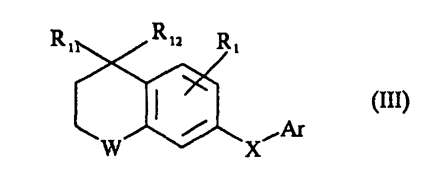 dans laquelle :
W représente S,
R₄, R₁₁, R₁₂ et X étant tels que définis à la revendication 13 et Ar représente le radical de formule (a).

15. Composés selon l'une quelconque des revendications 1 à 12, **caractérisés en ce qu'**ils répondent à la formule générale suivante : 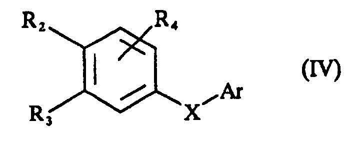 dans laquelle :
R₄ et X sont tels que définis à la revendication 13, Ar représente le radical de formule (a), l'un des radicaux R'₂ et/ou R'₃ représente un radical cycloalkyle mono ou polycyclique en C₅-C₁₀, l'autre représentant l'une des significations données pour R₂ et R₃ telles que définies à la revendication 1.

16. Composés selon l'une quelconque des revendications précédentes, **caractérisés en ce qu'**ils sont pris dans le groupe constitué par :
- 4 - (5, 5, 8, 8-tétraméthyl-5, 6, 7, 8-tétrahydronaphtalèn-2-ylsulfanyléthynyl) benzoate de méthyle,
- acide 4 - (5, 5, 8, 8-tétraméthyl-5, 6, 7, 8-tétrahydronaphtalèn-2-ylsulfanyléthynyl) benzoïque,
- 4 - (5, 5, 8, 8-tétraméthyl-5, 6, 7, 8-tétrahydronaphtalèn-2-ylsulfonyléthynyl) benzoate de méthyle,
- 4 - (5, 5, 8, 8-tétraméthyl-5, 6, 7, 8-tétrahydro-naphtalèn-2-yloxyéthynyl)-benzoate de méthyle,
- acide 4 - (5, 5, 8, 8-tétraméthyl-5, 6, 7, 8-tétrahydronaphtalèn-2-yloxyéthynyl) -benzoïque,
- 4 - (5, 5, 8, 8-tétraméthyl-5, 6, 7, 8-tétrahydronaphtalèn-2-ylsulfanyléthynyl) -benzoate de méthyle,
- acide 4 - (5, 5, 8, 8-tétraméthyl-5, 6, 7, 8-tétrahydronaphtalèn-2-ylsulfanyléthynyl) -benzoïque,
- 4 - (5, 5, 8, 8-tétraméthyl-5, 6, 7, 8-tétrahydronaphtalèn-2-ylsulfonyléthynyl) -benzoate de méthyle,
- acide 4- (5, 5, 8, 8-tétraméthyl-5, 6, 7, 8-tétrahydronaphtalèn-2-ylsulfonyléthynyl) -benzoïque,
- 4 - (5, 5, 8, 8-tétraméthyl-5, 6, 7, 8-tétrahydronaphtalèn-2-ylsulfinyléthynyl)-benzoate de méthyle,
- acide 4 - (5, 5, 8, 8-tétraméthyl-5, 6, 7, 8-tétrahydronaphtalèn-2-ylsulfinyléthynyl)-benzoïque,
- 4 - (5, 5, 8, 8-tétraméthyl-5, 6, 7, 8-tétrahydronaphtalèn-2-ylsélanyléthynyl) -benzoate de méthyle,
- acide 4 - (5, 5, 8, 8-tétraméthyl 5, 6, 7, 8-tétrahydronaphtalèn-2-ylsélanyléthynyl) -benzoïque,
- 2-hydroxy-4 - (5, 5, 8, 8-tétraméthyl-5, 6, 7, 8-tétrahydronaphtalèn-2-ylsélanyléthynyl) -benzoate de méthyle,
- acide 2-hydroxy-4- (5, 5, 8, 8-tétraméthyl-5, 6, 7, 8-tétrahydro-naphtalèn-2-ylsélanyléthynyl) -benzoïque,
- 6-(4-méthoxyméthoxy-phényléthynylsélanyl)-1,1,4,4-tétraméthyl-1, 2, 3, 4-tétrahydronaphtalène,
- 6-(5, 5, 8, 8-tétraméthyl - 5, 6, 7, 8-tétrahydronaphtanèn-2-ylsélanyléthynyl) -nicotinate d'éthyle,
- acide 6- (5, 5, 8, 8-tétraméthyl-5, 6, 7, 8-tétrahydronaphtalèn-2-ylsélanyléthynyl) -nicotinique,
- N-(4-hydroxy-phényl)-4-(5,5,8,8-tétraméthyl-5,6, 7, 8-tétrahydro-naphtalèn-2-ylsélanyléthynyl)-benzamide,
- 5-(5, 5, 8, 8-tétraméthyl-5, 6, 7, 8-tétrahydronaphtalèn-2-ylsélanyléthynyl)-pyridine-2-carboxylate de méthyle,
- 2-(-4-chloro-phénylsélanyléthynyl)-5, 5, 8, 8-tétraméthyl-5, 6, 7, 8-tétrahydronaphtalène,
- 4-(3, 5, 5, 8, 8-pentaméthyl-5, 6, 7, 8-tétrahydronaphtalèn-2-ylsélanyléthynyl) -benzoate de méthyle,
- acide 4-(3, 5, 5, 8, 8-pentaméthyl-5, 6, 7, 8-tétrahydronaphtalèn-2-ylsélanyléthynyl) -benzoïque,
- 2-hydroxy-4-(3, 5, 5, 8, 8-pentaméthyl-5, 6, 7, 8-tétrahdronaphtalèn-2-ylsélanyléthynyl) -benzoate de méthyle,
- acide 2-hydroxy-4-(3, 5, 5, 8, 8-pentaméthyl-5, 6, 7, 8-tétrahydro-naphtalèn-2-ylsélanyléthynyl)-benzoïque,
- 6-(3, 5, 5, 8, 8-pentaméthyl-5, 6, 7, 8-tétrahydronaphtalèn-2-ylsélanyléthynyl) -nicotinate d'éthyle,
- acide 6-(3, 5, 5, 8, 8-pentaméthyl-5, 6, 7, 8-tétrahydronaphtalèn-2-ylsélanyléthynyl) -nicotinique,
- N-(4-hydroxy-phényl)-6-(3, 5, 5, 8, 8-pentaméthyl-5, 6, 7, 8-tétrahydro-naphtalèn-2-ylsélanyléthynyl) -nicotinamide,
- N-butyl-6-(3, 5, 5, 8, 8-pentaméthyl-5, 6, 7, 8-tétrahydronaphtalèn-2-ylsélanyléthynyl) -nicotinamide,
- morpholin-4-yl-[6-(3, 5, 5, 8, 8-pentaméthyl-5, 6, 7, 8-tétrahydro-naphtalèn-2-ylsélanyléthynyl)-pyridine-3-yl]-méthanone,
- 5-(3,5,5,8,8-pentaméthyl-5,6,7,8-tétrahydronaphtalèn-2-ylsélanyléthynyl)-pyridine-2-carboxylate de méthyle,
- acide 5-(3,5,5,8,8-pentaméthyl-5,6, 7, 8-tétrahydronaphtalèn-2-ylsélanyléthynyl) -pyridine-2-carboxylique,
- [4-(5, 5, 8, 8-tétraméthyl-5, 6, 7, 8-tétrahydronaphtalèn-2-ylsélanyléthynyl)-phéhyl]-méthanol,
- 4-(5, 5, 8, 8-tétraméthyl-5,6, 7, 8-térrahydronaphtalèn-2-yléthynylsulfanyl)-benzoate de méthyle,
- 4-(5, 5, 8, 8-tétraméthyl-5, 6, 7, 8-tétrahydronaphtalèn-2-yléthynylsulfonyl) -benzoate de méthyle,
- 4-(5, 5, 8, 8,-tétraméthyl-5, 6, 7, 8-tétrahydronaphtalèn-2-yléthynylsulfinyl) -benzoate de méthyle,
- acide 4-(5, 5, 8, 8-tétraméthyl-5, 6, 7, 8-tétrahydronaphtalèn-2-yléthynylsulfinyl) -benzoate de méthyle,
- acide 4-(5, 5, 8, 8-tétraméthyl-5, 6, 7, 8-tétrahydronaphtalèn-2-yléthynylsulfanyl) -benzoïque,
- acide 4-(5, 5, 8, 8-tétraméthyl-5, 6, 7, 8-tétrahydronaphtalèn-2-yléthynylsulfonyl) -benzoïque,
- acide 4-(5, 5, 8, 8-tétraméthyl-5, 6, 7, 8-tétrahydronaphtalèn-2-yléthynylsulfinyl)-benzoïque,
- 4-(3, 5, 5, 8, 8-pentaméthyl-5, 6, 7, 8-tétrahydronaphtalèn-2-ylsélanyléthynyl) -phénol,
- 4-(4-hydroxy-5, 5, 8, 8-tétraméthyl-5, 6, 7, 8-tétrahydronaphtalèn-2-ylsélanyléthynyl) -benzoate d'éthyle,
- 4-(4-méthoxyméthoxy-5, 5, 8, 8-tétraméthyl-5, 6, 7, 8-tétrahydro-naphtalèn-2-ylsélanyléthynyl) -benzoate d'éthyle,
- acide 4-(4-méthoxyméthoxy-5, 5, 8, 8-tétraméthyl-5, 6, 7, 8-tétrahydro-naphtalèn-2-ylsélanyléthynyl) -benzoïque,
- acide 4-(4-pentyloxy-5, 5, 8, 8-tétraméthyl-5, 6, 7, 8-tétrahydronaphtalèn-2-ylsélanyléthynyl)-benzoïque,
- 4-(3-méthoxyméthoxy-5, 5, 8, 8-tétraméthyl-5, 6, 7, 8-tétrahydro-naphtalèn-2-ylsélanyléthynyl) -benzoate d'éthyle,
- 4-(3-méthoxyéthoxyméthoxy-5, 5, 8, 8-tétraméthyl-5, 6, 7, 8-tétrahydro-naphtalèn-2-ylsélanyléthynyl) -benzoate d'éthyle,
- acide 4-(3-méthoxyéthoxyméthoxy-5, 5, 8, 8-tétraméthyl-5, 6, 7, 8-tétrahydronaphtalèn-2-ylsélanyléthynyl) -benzoïque,
- acide 4-(3-méthoxyméthoxy-5, 5, 8, 8-tétraméthyl-5, 6, 7, 8-tétrahydro-naphtalèn-2-ylsélanyléthynyl) -benzoïque,
- 4-(3-pentyloxy-5, 5, 8, 8-tétraméthyl-5, 6, 7, 8-tétrahydronaphtalèn-2-ylsélanyléthynyl) -benzoate d'éthyle,
- acide 4-(3-pentyloxy-5, 5, 8, 8-tétraméthyl-5, 6, 7, 8-tétrahydronaphtalèn-2-ylsélanyléthynyl)-benzoïque,
- [4-(5, 5, 8, 8-tétraméthyl-5, 6, 7, 8-tétrahydronaphtalèn-2-ylsélanyléthynyl)-phényl]-carbaldéhyde,
- 4-(4, 4-diméthyl-thiochroman-8-ylsélanyléthynyl)-benzoate de méthyle,
- acide 4-(4, 4-diméthyl-thiochroman-8-ylsélanyléthynyl)-benzoïque,
- 4-(5, 5, 8, 8-tétraméthyl-5, 6, 7, 8-tétrahydronaphtalèn-8-ylsélanyléthynyl)-benzoate de méthyl,
- acide 4-(5, 5, 8, 8-tétraméthyl-5, 6, 7, 8-tétrahydronaphtalèn-8-ylsélanyléthynyl) -benzoïque,
- 4-[3-(1 adamantyl)-4-méthoxyphényl)-1-ylsélanyléthynyl] -benzoate de méthyle,
- acide 4-[3-(1-adamantyl)-4-méthoxyphényl)-1-ylsélanyléthynyl]-benzoïque,
- 4-[4-(1-adamantyl)-3-méthoxyphényl)-1-ylsélanyléthynyl]-benzoate de méthyl, et
- acide 4-[3-(1-adamantyl)-3-méthoxyphényl)-1-ylsélanyléthynyl]-benzoïque.

17. Composés selon l'une quelconque des revendications précédentes pour une utilisation comme médicament.

18. Composés selon la revendication 17 pour une utilisation comme médicament destiné au traitement des affections dermatologiques, des affections dermatologiques à composante inflammatoire et/ou immuno-allergique du type rhumatismale ou respiratoire, des affections cardio-vasculaires et des troubles ophtalmologiques.

19. Utilisation de l'un au moins des composés tels que définis selon l'une quelconque des revendications 1 à 16 pour la préparation d'un médicament destiné au traitement des affections dermatologiques, des affections dermatologiques à composante inflammatoire et/ou immuno-allergique du type rhumatismale ou respiratoire, des affections cardio-vasculaires et des troubles ophtalmologiques.

20. Composition pharmaceutique, **caractérisée par le fait qu'**elle comprend, dans un support pharmaceutiquement acceptable, au moins un composé tel que défini selon l'une quelconque des revendications 1 à 16.

21. Composition selon la revendication 20, **caractérisée en ce que** la concentration en au moins un composé selon l'une des revendications 1 à 16 est comprise entre 0,001 % et 5 % en poids par rapport au poids total de la composition.

22. Composition cosmétique, **caractérisée en ce qu'**elle contient, dans un support cosmétiquement acceptable, au moins un composé tel que défini selon l'une quelconque des revendications 1 à 16.

23. Composition selon la revendication 22, **caractérisée en ce que** la concentration en au moins un composé selon l'une quelconque des revendication 1 à 16 est comprise entre 0,001 % et 3 % en poids par rapport au poids total de la composition.

24. Utilisation d'une composition cosmétique telle que définie selon l'une quelconque des revendications 21 ou 23 pour l'hygiène corporelle ou capillaire.

## Patentansprüche

1. Biaromatische Verbindung, verbunden über eine Heteroethinylenbindung, **dadurch gekennzeichnet, dass** sie der folgenden allgemeinen Formel (I) entsprechen: 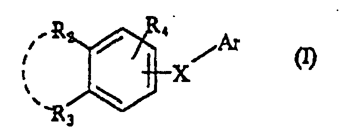 worin:
Ar für einen Rest steht, ausgewählt aus den folgenden Formeln (a) und (b): 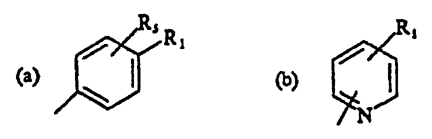
R₁ für ein Halogenatom, -CH₃, -CH₂-OR₇, -OR₇ oder -COR₈ steht,
R₂ und R₃, gleich oder verschieden sind und für H, lineares oder verzweigtes C₁-C₂₀-Alkyl, C₃-C₁₂-Cycloalkyl oder -OR₇ stehen, wobei mindestens einer der Reste R₂ und R₃ lineares oder verzweigtes C₁-C₂₀-Alkyl oder C₃-C₁₀-Cycloalkyl darstellt, oder
worin R₂ und R₃ zusammengenommen einen 5- oder 6-gliedrigen Ring bilden, der mindestens ein Methyl tragen kann und/oder durch ein Heteroatom unterbrochen sein kann, ausgewählt unter O oder S,
R₄ und R₅ für H, ein Halogenatom, lineares oder verzweigtes C₁-C₂₀-Alkyl, -OR₇ oder einen Polyetherrest stehen,
R₇ für H, lineares oder verzweigtes C₁-C₁₀-Alkyl oder -COR₉ steht,
R₈ für H, lineares oder verzweigtes C₁-C₁₀-Alkyl, -OR₁₀ oder 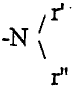 steht,
R₉ für lineares oder verzweigtes C₁-C₁₀-Alkyl steht,
R₁₀ für H, lineares oder verzweigtes C₁-C₂₀-Alkyl, Mono- oder Polyhydroxyalkyl, Allyl, Aryl oder Aralkyl steht,
r' und r", die gleich oder verschieden sind, für H, C₁-C₁₀-Alkyl, Mono- oder Polyhydroxyalkyl, Aryl stehen oder zusammen mit dem Stickstoffatom einen Heterozyklus bilden,
X für einen divalenten Rest steht, der von rechts nach links oder umgekehrt die folgende Formel aufweist: 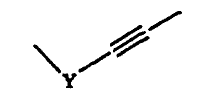
worin:
Y für S(O)ₙ oder Se(O)_{n'} steht,
worin n und n' 0, 1 oder 2 sind,
mit der Maßgabe, dass dann, wenn Ar ein Rest der Formel (a) oben ist, in der R₁ = -CH₃, ein Halogenatom oder ein Rest -OR₇ ist und R₅ = H ist, sich einer der Reste R₂ oder R₃ von -CH₃ unterscheidet,
und die Salze der Verbindungen der Formel (I), wenn R₁ eine Carbonsäurefunktion darstellt, wie auch die optischen Isomere der Verbindungen der Formel (I).

2. Verbindungen gemäß Anspruch 1, **dadurch gekennzeichnet, dass** sie in Form eines Salzes eines Alkalimetalls oder Erdalkalimetalls oder auch von Zink oder einem organischen Amin vorliegen.

3. Verbindungen gemäß einem der Ansprüche 1 oder 2, **dadurch gekennzeichnet, dass** der C₁-C₁₀-Alkylrest ausgewählt ist aus der Gruppe, bestehend aus den Resten Methyl, Ethyl, Isopropyl, Butyl, tert.-Butyl, Hexyl, 2-Ethylhexyl oder Octyl.

4. Verbindungen gemäß einem der vorstehenden Ansprüche, **dadurch gekennzeichnet, dass** der lineare oder verzweigte C₁-C₂₀-Alkylrest ausgewählt ist aus der Gruppe, bestehend aus den Resten Methyl, Ethyl, Propyl, 2-Ethylhexyl, Octyl, Dodecyl, Hexadecyl und Octadecyl.

5. Verbindungen gemäß einem der vorstehenden Ansprüche, **dadurch gekennzeichnet, dass** der C₃-C₁₂-Cycloalkylrest ausgewählt ist aus der Gruppe, bestehend aus den Resten Cyclopropyl, Cyclopentyl, Cyclohexyl, 1-Methylcyclohexyl oder 1-Adamantyl.

6. Verbindungen gemäß einem der vorstehenden Ansprüche, **dadurch gekennzeichnet, dass** der Polyetherrest ausgewählt ist aus der Gruppe, bestehend aus den Resten Methoxymethoxy, Methoxyethoxy und Methoxyethoxymethoxy.

7. Verbindungen gemäß einem der vorstehenden Ansprüche, **dadurch gekennzeichnet, dass** der Monohydroxyalkylrest ausgewählt ist aus der Gruppe, bestehend aus den Resten 2-Hydroxyethyl, 2-Hydroxypropyl oder 3-Hydroxypropyl.

8. Verbindungen gemäß einem der vorstehenden Ansprüche, **dadurch gekennzeichnet, dass** der Polyhydroxyalkylrest ausgewählt ist aus der Gruppe, bestehend aus den Resten 2,3-Dihydroxypropyl, 2,3,4-Trihydroxybutyl, 2,3,4,5-Tetrahydroxypentyl oder dem Pentaerythritrest.

9. Verbindungen gemäß einem der vorstehenden Ansprüche, **dadurch gekennzeichnet, dass** der Arylrest ein Phenylrest ist.

10. Verbindungen gemäß einem der vorstehenden Ansprüche, **dadurch gekennzeichnet, dass** der Aralkylrest ausgewählt ist aus der Gruppe, bestehend aus den Resten Benzyl oder Phenethyl.

11. Verbindungen gemäß einem der vorstehenden Ansprüche, **dadurch gekennzeichnet, dass** der heterocyclische Rest ausgewählt ist aus der Gruppe, bestehend aus den Resten Piperidino, Morpholino, Pyrrolidino, Piperazino oder Piperazino, substituiert in Position 4 mit einem C₁-C₆-Alkyl oder einem Mono- oder Polyhydroxyalkyl.

12. Verbindungen gemäß einem der vorstehenden Ansprüche, **dadurch gekennzeichnet, dass** das Halogenatom ausgewählt ist aus der Gruppe, bestehend aus Fluor, Chlor und Brom.

13. Verbindungen gemäß einem der vorstehenden Ansprüche, **dadurch gekennzeichnet, dass** sie der folgenden allgemeinen Formel entsprechen: 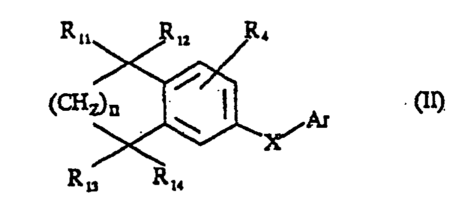 worin:
Ar für einen Rest der Formel steht: 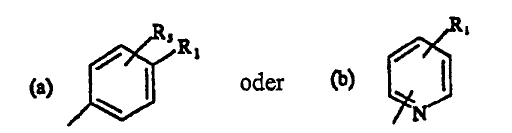
worin R₁, R₄, R₅ und X wie in Anspruch 1 definiert sind,
R₁₁, R₁₂, R₁₃ und R₁₄, gleich oder verschieden, für H oder -CH₃ stehen und
n 1 oder 2 ist.

14. Verbindungen gemäß einem der Ansprüche 1 bis 12, **dadurch gekennzeichnet, dass** sie der folgenden allgemeinen Formel entsprechen: 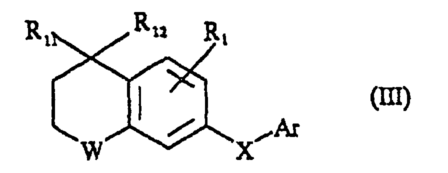 worin:
W für S steht,
R₄, R₁₁, R₁₂ und X wie in Anspruch 13 definiert sind und worin Ar für den Rest der Formel (a) steht.

15. Verbindungen gemäß einem der Ansprüche 1 bis 12, **dadurch gekennzeichnet, dass** sie der folgenden allgemeinen Formel entsprechen: 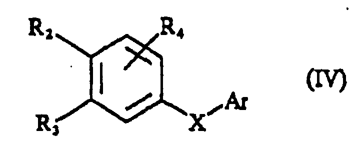 worin:
R₄ und X wie in Anspruch 13 definiert sind, Ar für den Rest der Formel (a) steht, einer der Reste R'₂ und/oder R'₃ für einen mono- oder polycyclischen C₅-C₁₀-Cycloalkylrest steht, wobei der jeweils andere eine der für R₂ und R₃ gegebenen Bedeutungen hat, wie sie in Anspruch 1 definiert sind.

16. Verbindungen nach einem der vorstehenden Ansprüche, **dadurch gekennzeichnet, dass** sie aus der Gruppe sind, bestehend aus:
- 4-(5,5,8,8-Tetramethyl-5,6,7,8-tetrahydronaphtalin-2-ylsulfanylethinyl)-benzoesäuremethylester,
- 4-(5,5,8,8-Tetramethyl-5,6,7,8-tetrahydronaphtalin-2-ylsulfanylethinyl)-benzoesäure,
- 4-(5,5,8,8-Tetramethyl-5,6,7,8-tetrahydronaphtalin-2-ylsulfonylethinyl)-benzoesäuremethylester,
- 4-(5,5,8,8-Tetramethyl-5,6,7,8-tetrahydronaphtalin-2-yloxyethinyl)-benzoesäuremethylester,
- 4-(5,5,8,8-Tetramethyl-5,6,7,8-tetrahydronaphtalin-2-yloxyethinyl)-benzoesäure,
- 4-(5,5,8,8-Tetramethyl-5,6,7,8-tetrahydronaphtalin-2-ylsulfanylethinyl)-benzoesäuremethylester,
- 4-(5,5,8,8-Tetramethyl-5,6,7,8-tetrahydronaphtalin-2-ylsulfanylethinyl)-benzoesäure,
- 4-(5,5,8,8-Tetramethyl-5,6,7,8-tetrahydronaphtalin-2-ylsulfonylethinyl)-benzoesäuremethylester,
- 4-(5,5,8,8-Tetramethyl-5,6,7,8-tetrahydronaphtalin-2-ylsulfonylethinyl)-benzoesäure,
- 4-(5,5,8,8-Tetramethyl-5,6,7,8-tetrahydronaphtalin-2-ylsulfinylethinyl)-benzoesäuremethylester,
- 4-(5,5,8,8-Tetramethyl-5,6,7,8-tetrahydronaphtalin-2-ylsulfinylethinyl)-benzoesäure,
- 4-(5,5,8,8-Tetramethyl-5,6,7,8-tetrahydronaphtalin-2-ylselanylethinyl)-benzoesäuremethylester,
- 4-(5,5,8,8-Tetramethyl-5,6,7,8-tetrahydronaphtalin-2-ylselanylethinyl)-benzoesäure,
- 2-Hydroxy-4-(5,5,8,8-tetramethyl-5,6,7,8-tetrahydronaphtalin-2-ylselanylethinyl)-benzoesäuremethylester,
- 2-Hydroxy-4-(5,5,8,8-tetramethyl-5,6,7,8-tetrahydronaphtalin-2-ylselanylethinyl)-benzoesäure,
- 6-(4-Methoxymethoxyphenylethinylselanyl)-1,1,4,4-tetramethyl-1,2,3,4-tetrahydronaphtalin,
- 6-(5,5,8,8-Tetramethyl-5 ,6,7,8-tetrahydronaphtaniii-2-ylselanylethinyl)-nicotinsäureethylester,
- 6-(5,5,8,8-Tetamethyl-5,6,7,8-tetrahydronaphtalin-2-ylselanylethinyl)-nicotinsäure,
- N-(4-Hydroxyphenyl)-4-(5,5,8,8-tetramethyl-5,6,7,8-tetrahydronaphtalin-2-ylselanylethinyl)-benzamid,
- 5-(5,5,8,8-Tetramethyl-5,6,7,8-tetrahydronaphtalin-2-ylselanylethinyl)-pyridin-2-carbonsäuremethylester,
- 2-(-4-Chlorphenylselanylethinyl)-5,5,8,8-tetramethyl-5,6,7,8-tetrahydronaphtalin,
- 4-(3,5,5,8,8-Pentamethyl-5,6,7,8-tetrahydronaphtalin-2-ylselanylethinyl)-benzoesäuremethylester,
- 4-(3,5,5,8,8-Pentamethyl-5,6,7,8-tetrahydronaphtalin-2-ylselanylethinyl)-benzoesäure,
- 2-Hydroxy-4-(3,5,5,8,8-pentamethyl-5,6,7,8-tetrahydronaphtalin-2-ylselanylethinyl)-benzoesäuremethylester,
- 2-Hydroxy-4-(3,5,5,8,8-pentamethyl-5,6,7,8-tetrahydronaphtalin-2-ylselanylethinyl)-benzoesäure,
- 6-(3,5,5,8,8-Pentamethyl-5,6,7,8-tetrahydronaphtalin-2-ylselanylethinyl)-nicotinsäureethylester,
- 6-(3,5,5,8,8-Pentamethyl-5,6,7,8-tetrahydronaphtalin-2-ylselanylethinyl)-nicotinsäure,
- N-(4-Hydroxyphenyl)-6-(3,5,5,8,8-pentamethyl-5,6,7,8-tetrahydronaphtalin-2-ylselanylethinyl)-nicotinamid,
- N-Butyl-6-(3,5,5,8,8-pentamethyl-5,6,7,8-tetrahydronaphtalin-2-ylselanylethinyl)-nicotinamid,
- Morpholin-4-yl-[6-(3,5,5,8,8-pentamethyl-5,6,7,8-tetrahydronaphtalin-2-ylselanylethinyl)-pyridin-3-yl]-methanon,
- 5-(3,5,5,8,8-Pentamethyl-5,6,7,8-tetrahydronaphtalin-2-ylselanylethinyl)-pyridin-2-carbonsäuremethylester,
- 5-(3,5,5,8,8-Pentamethyl-5,6,7,8-tetrahydronaphtalin-2-ylselanylethinyl)-pyridin-2-carbonsäure,
- [4-(5,5,8,8-Tetramethyl-5,6,7,8-tetrahydronaphtalin-2-ylselanylethinyl)-phenyl]-methanol,
- 4-(5,5,8,8-Tetramethyl-5,6,7,8-tetrahydronaphtalin-2-ylethinylsulfanyl)-benzoesäuremethylester,
- 4-(5,5,8,8-Tetramethyl-5,6,7,8-tetrahydronaphtalin-2-ylethinylsulfonyl)-benzoesäuremethylester,
- 4-(5,5,8,8-Tetramethyl-5,6,7,8-tetrahydronaphtalin-2-ylethinylsulfinyl)-benzoesäuremethylester,
- 4-(5,5,8,8-Tetramethyl-5,6,7,8-tetrahydronaphtalin-2-ylethinylsulfinyl)-benzoesäure-(methylester),
- 4-(5,5,8,8-Tetramethyl-5,6,7,8-tetrahydronaphtalin-2-ylethinylsulfanyl)-benzoesäure,
- 4-(5,5,8,8-Tetramethyl-5,6,7,8-tetrahydronaphtalin-2-ylethinylsulfonyl)-benzoesäure,
- 4-(5,5,8,8-Tetramethyl-5,6,7,8-tetrahydronaphtalin-2-ylethinylsulfinyl)-benzoesäure,
- 4-(3,5,5,8,8-Pentamethyl-5,6,7,8-tetrahydronaphtalin-2-ylselanylethinyl)-phenol,
- 4-(4-Hydroxy-5,5,8,8-tetramethyl-5,6,7,8-tetrahydronaphtalin-2-ylselanylethinyl)-benzoesäureethylester,
- 4-(4-Methoxymethoxy-5,5,8,8-tetramethyl-5,6,7,8-tetrahydronaphtalin-2-ylselanylethinyl)-benzoesäureethylester,
- 4-(4-Methoxymethoxy-5,5,8,8-tetramethyl-5,6,7,8-tetrahydronaphtalin-2-ylselanylethinyl)-benzoesäure,
- 4-(4-Pentyloxy-5,5,8,8-tetramethyl-5,6,7,8-tetrahydronaphtalin-2-ylselanylethinyl)-benzoesäure,
- 4-(3-Methoxymethoxy-5,5,8,8-tetramethyl-5,6,7,8-tetrahydronaphtalin-2-ylselanylethinyl)-benzoesäureethylester,
- 4-(3-Methoxyethoxymethoxy-5,5,8,8-tetramethyl-5,6,7,8-tetrahydronaphtalin-2-ylselanylethinyl)-benzoesäureethylester,
- 4-(3-Methoxyethoxymethoxy-5,5,8,8-tetramethyl-5,6,7,8-tetrahydronaphtalin-2-ylselanylethinyl)-benzoesäure,
- 4-(3-Methoxymethoxy-5,5,8,8-tetramethyl-5,6,7,8-tetrahydronaphtalin-2-ylselanylethinyl)-benzoesäure,
- 4-(3-Pentyloxy-5,5,8,8-tetramethyl-5,6,7,8-tetrahydronaphtalin-2-ylselanylethinyl)-benzoesäureethylester,
- 4-(3-Pentyloxy-5,5,8,8-tetramethyl-5,6,7,8-tetrahydronaphtalin-2-ylselanylethinyl)-benzoesäure,
- [4-(5,5,8,8-Tetramethyl-5,6,7,8-tetrahydronaphtalin-2-ylselanylethinyl)-phenyl]-carbaldehyd,
- 4-(4,4-Dimethylthiochroman-8-ylselanylethinyl)-benzoesäuremethylester,
- 4-(4,4-Dimethylthiochroman-8-ylselanylethinyl)-benzoesäure,
- 4-(5,5,8,8-Tetramethyl-5,6,7,8-tetrahydronaphtalin-8-ylselanylethinyl)-benzoesäuremethylester,
- 4-(5,5,8,8-Tetramethyl-5,6,7,8-tetrahydronaphtalin-8-ylselanylethinyl)-benzoesäure,
- 4-[3-(1-Adamantyl)-4-methoxyphenyl)-1-ylselanylethinyl]-benzoesäuremethylester,
- 4-[3-(1-Adamantyl)-4-methoxyphenyl)-1-ylselanylethinyl]-benzoesäure,
- 4-[4-(1-Adamantyl)-3-methoxyphenyl)-1-ylselanylethinyl]-benzoesäuremethylester,
- 4-[3-(1-Adamantyl)-3-methoxyphenyl)-1-ylselanylethinyl]-benzoesäure.

17. Verbindungen nach einem der vorstehenden Ansprüche für die Verwendung als Medikament.

18. Verbindungen gemäß Anspruch 17 für eine Verwendung als Medikament, bestimmt zur Behandlung von dermatologischen Störungen, dermatologischen Störungen mit entzündlicher und/oder immunallergischer Komponente vom rheumatischen oder respiratorischen Typ, von kardiovaskulären Störungen und ophtalmologischen Problemen.

19. Verwendung mindestens einer der Verbindungen, wie sie in einem der Ansprüche 1 bis 16 definiert sind, für die Herstellung eines Medikaments, bestimmt zur Behandlung von dermatologischen Störungen, dermatologischen Störungen mit entzündlicher und/oder immunallergischer Komponente vom rheumatischen oder respiratorischen Typ, von kardiovaskulären Störungen und ophtalmologischen Problemen.

20. Pharmazeutische Zubereitung, **dadurch gekennzeichnet, dass** sie in einem pharmazeutisch annehmbaren Träger mindestens eine Verbindung enthält, wie sie in einem der Ansprüche 1 bis 16 definiert ist.

21. Zubereitung gemäß Anspruch 20, **dadurch gekennzeichnet, dass** die Konzentration der mindestens einen Verbindung gemäß einem der Ansprüche 1 bis 16 zwischen 0,001 Gew.-% und 5 Gew.-% liegt, bezogen auf das Gesamtgewicht der Zubereitung.

22. Kosmetische Zubereitung, **dadurch gekennzeichnet, dass** sie in einem kosmetisch annehmbaren Träger mindestens eine Verbindung enthält, wie sie in einem der Ansprüche 1 bis 16 definiert ist.

23. Zubereitung gemäß Anspruch 22, **dadurch gekennzeichnet, dass** die Konzentration der mindestens einen Verbindung gemäß einem der Ansprüche 1 bis 16 zwischen 0,001 Gew.-% und 3 Gew.-% liegt, bezogen auf das Gesamtgewicht der Zubereitung.

24. Verwendung einer kosmetischen Zubereitung, wie sie in einem der Ansprüche 21 oder 23 definiert ist, für die Körper- oder Haarpflege.

## Claims

1. Bi-aromatic compounds linked via a heteroethynylene bond, **characterized in that** they correspond to the general formula (I) below: 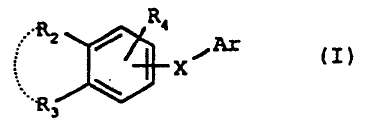 in which:
Ar represents a radical chosen from the formulae (a) and (b) below: 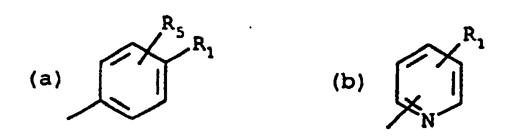
R₁ represents a halogen atom, -CH₃, -CH₂-OR₇, -OR₇ or -COR₈,
R₂ and R₃, which may be identical or different, represent H, linear or branched C₁-C₂₀ alkyl, C₃-C₁₂ cycloalkyl or -OR₇, at least one from among R₂ and R₃ being linear or branched C₁-C₂₀ alkyl or C₃-C₁₀ cycloalkyl, or
R₂ and R₃, taken together, form a 5- or 6-membered ring, which can comprise at least one methyl and/or which can be interrupted by a hetero atom chosen from O and S,
R₄ and R₅ represent H, a halogen atom, linear or branched C₁-C₂₀ alkyl, -OR₇ or a polyether radical,
R₇ represents H, linear or branched C₁-C₁₀ alkyl or -COR₉,
R₈ represents H, linear or branched C₁-C₁₀ alkyl, -OR₁₀ or 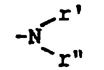
R₉ represents linear or branched C₁-C₁₀ alkyl,
R₁₀ represents H, linear or branched C₁-C₂₀ alkyl, mono- or polyhydroxyalkyl, allyl, aryl or aralkyl,
r' and r", which may be identical or different, represent H, C₁-C₁₀ alkyl, mono- or polyhydroxyalkyl, aryl, or, taken together with the nitrogen atom, form a heterocycle,
X represents a divalent radical which, from right to left or vice-versa, has the formula: 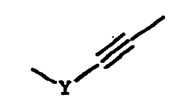
in which:
Y represents S(O)ₙ or Se(O)_{n'},
n and n' being 0, 1 or 2,
with the proviso that when Ar is a radical of formula (a) above, in which R₁=-CH₃, a halogen atom or a radical -OR₇ and R₅=H, then one of the radicals R₂ or R₃ is other than -CH₃,
and the salts of the compounds of formula (I) when R₁ represents a carboxylic acid function, as well as the optical isomers of the said compounds of formula (I).

2. Compounds according to Claim 1, **characterized in that** they are in the form of a salt of an alkali metal or alkaline-earth metal, or alternatively of zinc or of an organic amine.

3. Compounds according to either of Claims 1 and 2, **characterized in that** the C₁-C₁₀ alkyl radical is chosen from the group consisting of the methyl, ethyl, isopropyl, butyl, tert-butyl, hexyl, 2-ethylhexyl and octyl radicals.

4. Compounds according to any one of the preceding claims, **characterized in that** the linear or branched C₁-C₂₀ alkyl radical is chosen from the group consisting of the methyl, ethyl, propyl, 2-ethylhexyl, octyl, dodecyl, hexadecyl and octadecyl radicals.

5. Compounds according to any one of the preceding claims, **characterized in that** the C₃-C₁₂ cycloalkyl radical is chosen from the group consisting of the cyclopropyl, cyclopentyl, cyclohexyl, 1-methylcyclohexyl and 1-adamantyl radicals.

6. Compounds according to any one of the preceding claims, **characterized in that** the polyether radical is chosen from the group consisting of the methoxymethoxy, methoxyethoxy and methoxyethoxymethoxy radicals.

7. Compounds according to any one of the preceding claims, **characterized in that** the monohydroxyalkyl radical is chosen from the group consisting of the 2-hydroxyethyl, 2-hydroxypropyl and 3-hydroxypropyl radicals.

8. Compounds according to any one of the preceding claims, **characterized in that** the polyhydroxyalkyl radical is chosen from the group consisting of the 2,3-dihydroxypropyl, 2,3,4-trihydroxybutyl and 2,3,4,5-tetrahydroxypentyl radicals and the pentaerythritol residue.

9. Compounds according to any one of the preceding claims, **characterized in that** the aryl radical is a phenyl radical.

10. Compounds according to any one of the preceding claims, **characterized in that** the aralkyl radical is chosen from the group consisting of the benzyl and phenethyl radicals.

11. Compounds according to any one of the preceding claims, **characterized in that** the heterocyclic radical is chosen from the group consisting of the piperidino, morpholino, pyrrolidino and piperazino radicals, or the piperazine radical substituted in position 4 with a C₁-C₆ alkyl or a mono- or polyhydroxyalkyl.

12. Compounds according to any one of the preceding claims, **characterized in that** the halogen atom is chosen from the group consisting of fluorine, chlorine and bromine.

13. Compounds according to any one of the preceding claims, **characterized in that** they correspond to the following general formula: 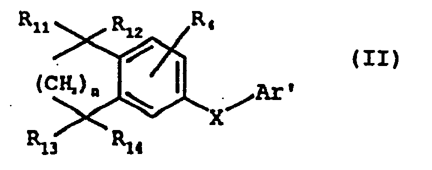 in which:
Ar' represents a radical of formula: 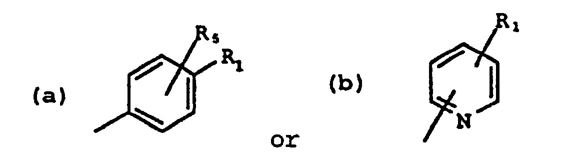
R₁, R₄, R₅ and X being as defined in Claim 1,
R₁₁, R₁₂, R₁₃ and R₁₄, which may be identical or different, represent H or -CH₃, and
n is 1 or 2.

14. Compounds according to any one of Claims 1 to 12, **characterized in that** they correspond to the following general formula 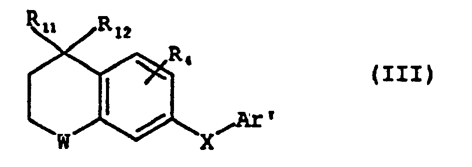 in which:
W represents S,
R₄, R₁₁, R₁₂ and X being as defined in Claim 13 and Ar represents the radical of formula (a).

15. Compounds according to any one of Claims 1 to 12, **characterized in that** they correspond to the following general formula: 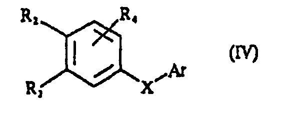 in which:
R₄ and X are as defined in Claim 13, Ar represents the radical of formula (a), one of the radicals R'₂ and/or R'₃ represents a mono- or polycyclic C₅-C₁₀ cycloalkyl radical, the other representing one of the meanings given for R₂ and R₃ as defined in Claim 1.

16. Compounds according to any one of the preceding claims, **characterized in that** they are taken from the group consisting of:
- Methyl 4-(5,5,8,8,-tetramethyl-5,6,7,8-tetrahydro-2-naphthylsulphanylethynyl)benzoate,
- 4-(5,5,8,8,-Tetramethyl-5,6,7,8-tetrahydro-2-naphthylsulphanylethynyl)benzoic acid,
- Methyl 4-(5,5,8,8,-tetramethyl-5,6,7,8-tetrahydro-2-naphthylsulphonylethynyl)benzoate,
- Methyl 4-(5,5,8,8,-tetramethyl-5,6,7,8-tetrahydro-2-naphthyloxyethynyl)benzoate,
- 4-(5,5,8,8,-Tetramethyl-5,6,7,8-tetrahydro-2-naphthyloxyethynyl)benzoic acid,
- Methyl 4-(5,5,8,8,-tetramethyl-5,6,7,8-tetrahydro-2-naphthylsulphanylethynyl)benzoate,
- 4-(5,5,8,8,-Tetramethyl-5,6,7,8-tetrahydro-2-naphthylsulphanylethynyl)benzoic acid,
- Methyl 4-(5,5,8,8,-tetramethyl-5,6,7,8-tetrahydro-2-naphthylsulphonylethynyl)benzoate,
- 4-(5,5,8,8,-Tetramethyl-5,6,7,8-tetrahydro-2-naphthylsulphonylethynyl)benzoic acid,
- Methyl 4-(5,5,8,8,-tetramethyl-5,6,7,8-tetrahydro-2-naphthylsulphinylethynyl)benzoate,
- 4-(5,5,8,8,-Tetramethyl-5,6,7,8-tetrahydro-2-naphthylsulphinylethynyl)benzoic acid,
- Methyl 4-(5,5,8,8,-tetramethyl-5,6,7,8-tetrahydro-2-naphthylselanylethynyl)benzoate,
- 4-(5,5,8,8,-Tetramethyl-5,6,7,8-tetrahydro-2-naphthylselanylethynyl)benzoic acid,
- Methyl 2-hydroxy-4-(5,5,8,8,-tetramethyl-5,6,7,8-tetrahydro-2-naphthylselanylethynyl)benzoate,
- 2-Hydroxy-4-(5,5,8,8,-tetramethyl-5,6,7,8-tetrahydro-2-naphthylselanylethynyl)benzoic acid,
- 6-(4-Methoxymethoxyphenylethynylselanyl)-1,1,4,4-tetramethyl-1,2,3,4-tetrahydronaphthalene,
- Ethyl 6-(5,5,8,8,-tetramethyl-5,6,7,8-tetrahydro-2-naphthylselanylethynyl)nicotinate,
- 6-(5,5,8,8,-Tetramethyl-5,6,7,8-tetrahydro-2-naphthylselanylethynyl)nicotinic acid,
- N-(4-Hydroxyphenyl)-4-(5,5,8,8,-tetramethyl-5,6,7,8-tetrahydro-2-naphthylselanylethynyl)benzamide,
- Methyl 5-(5,5,8,8,-tetramethyl-5,6,7,8-tetrahydro-2-naphthylselanylethynyl)-2-pyridine carboxylate,
- 2-(4-Chlorophenylselanylethynyl)-5,5,8,8-tetramethyl-5,6,7,8-tetrahydronaphthalene,
- Methyl 4-(3,5,5,8,8-pentamethyl-5,6,7,8-tetrahydro-2-naphthylselanylethynyl)benzoate,
- 4-(3,5,5,8,8-Pentamethyl-5,6,7,8-tetrahydro-2-naphthylselanylethynyl)benzoic acid,
- Methyl 2-hydroxy-4-(3,5,5,8,8-pentamethyl-5,6,7,8-tetrahydro-2-naphthylselanylethynyl)benzoate,
- 2-Hydroxy-4-(3,5,5,8,8-pentamethyl-5,6,7,8-tetrahydro-2-naphthylselanylethynyl)benzoic acid,
- Ethyl 6-(3,5,5,8,8-pentamethyl-5,6,7,8-tetrahydro-2-naphthylselanylethynyl)nicotinate,
- 6-(3,5,5,8,8-Pentamethyl-5,6,7,8-tetrahydro-2-naphthylselanylethynyl)nicotinic acid,
- N-(4-Hydroxyphenyl)-6-(3,5,5,8,8-pentamethyl-5,6,7,8-tetrahydro-2-naphthylselanylethynyl)nicotin-amide,
- N-Butyl-6-(3,5,5,8,8-pentamethyl-5,6,7,8-tetrahydro-2-naphthylselanylethynyl)nicotinamide,
- Morpholin-4-yl-[6-(3,5,5,8,8,-pentamethyl-5,6,7,8-tetrahydro-2-naphthylselanylethynyl)-3-pyridyl]methanone,
- Methyl 5-(3,5,5,8,8-pentamethyl-5,6,7,8-tetrahydro-2-naphthylselanylethynyl)pyridine-2-carboxylate,
- 5-(3,5,5,8,8-Pentamethyl-5,6,7,8-tetrahydro-2-naphthylselanylethynyl)pyridine-2-carboxylic acid,
- [4-(5,5,8,8-Tetramethyl-5,6,7,8-tetrahydro-2-naphthylselanylethynyl)phenyl]methanol,
- Methyl 4-(5,5,8,8-tetramethyl-5,6,7,8-tetrahydro-2-naphthylethynylsulphanyl)benzoate,
- Methyl 4-(5,5,8,8-tetramethyl-5,6,7,8-tetrahydro-2-naphthylethynylsulphonyl)benzoate,
- Methyl 4-(5,5,8,8-tetramethyl-5,6,7,8-tetrahydro-2-naphthylethynylsulphinyl)benzoate,
- 4-(5,5,8,8-Tetramethyl-5,6,7,8-tetrahydro-2-naphthylethynylsulphanyl)benzoic acid,
- 4-(5,5,8,8-Tetramethyl-5,6,7,8-tetrahydro-2-naphthylethynylsulphonyl)benzoic acid,
- 4-(5,5,8,8-Tetramethyl-5,6,7,8-tetrahydro-2-naphthylethynylsulphinyl)benzoic acid,
- 4-(3,5,5,8,8-Pentamethyl-5,6,7,8-tetrahydro-2-naphthylselanylethynyl)phenol,
- Ethyl 4-(4-hydroxy-5,5,8,8-tetramethyl-5,6,7,8-tetrahydro-2-naphthylselanylethynyl)benzoate,
- Ethyl 4-(4-methoxymethoxy-5,5,8,8-tetramethyl-5,6,7,8-tetrahydro-2-naphthylselanylethynyl)benzoate,
- 4-(4-Methoxymethoxy-5,5,8,8-tetramethyl-5,6,7,8-tetrahydro-2-naphthylselanylethynyl)benzoic acid,
- 4-(4-Pentyloxy-5,5,8,8-tetramethyl-5,6,7,8-tetrahydro-2-naphthylselanylethynyl)benzoic acid,
- Ethyl 4-(3-methoxymethoxy-5,5,8,8-tetramethyl-5,6,7,8-tetrahydro-2-naphthylselanylethynyl)benzoate,
- Ethyl 4-(3-methoxyethoxymethoxy-5,5,8,8-tetramethyl-5,6,7,8-tetrahydro-2-naphthylselanylethynyl)benzoate,
- 4-(3-Methoxyethoxymethoxy-5,5,8,8-tetramethyl-5,6,7,8-tetrahydro-2-naphthylselanylethynyl)benzoic acid,
- 4- (3-Methoxymethoxy-5,5,8,8-tetramethyl-5,6,7,8-tetrahydro-2-naphthylselanylethynyl)benzoic acid,
- Ethyl 4-(3-pentyloxy-5,5,8,8-tetramethyl-5,6,7,8-tetrahydro-2-naphthylselanylethynyl)benzoate,
- 4-(3-Pentyloxy-5,5,8,8-tetramethyl-5,6,7,8-tetrahydro-2-naphthylselanylethynyl)benzoic acid,
- [4-(5,5,8,8-Tetramethyl-5,6,7,8-tetrahydro-2-naphthylselanylethynyl)phenyl]carbaldehyde,
- Methyl 4-(4,4-dimethylthiochroman-8-ylselanylethynyl)benzoate,
- 4-(4,4-Dimethylthiochroman-8-ylselanylethynyl)benzoic acid,
- Methyl 4-(5,5,8,8-tetramethyl-5,6,7,8-tetrahydro-8-naphthylselanylethynyl)benzoate,
- 4-(5,5,8,8-Tetramethyl-5,6,7,8-tetrahydro-8-naphthylselanylethynyl)benzoic acid,
- Methyl 4-[3-(1-adamantyl)-4-methoxyphenyl)-1-ylselanylethynyl]benzoate,
- 4-[3-(1-Adamantyl)-4-methoxyphenyl)-1-ylselanylethynyl]benzoic acid,
- Methyl 4-[4-(1-adamantyl)-3-methoxyphenyl)-1-ylselanylethynyl]benzoate, and
- 4-[3-(1-Adamantyl)-3-methoxyphenyl)-1-ylselanylethynyl]benzoic acid.

17. Compounds according to any one of the preceding claims, for use as a medicinal product.

18. Compounds according to Claim 17, for use as a medicinal product intended for the treatment of dermatological complaints, dermatological complaints with an inflammatory and/or immunoallergic component of the rheumatic or respiratory type, cardiovascular complaints and opthalmological disorders.

19. Use of at least one of the compounds as defined according to any one of Claims 1 to 16, for the preparation of a medicinal product intended for the treatment of dermatological complaints, dermatological complaints with an inflammatory and/or immunoallergic component of the rheumatic or respiratory type, cardiovascular complaints and opthalmological disorders.

20. Pharmaceutical composition, **characterized in that** it comprises, in a pharmaceutically acceptable support, at least one compound as defined according to any one of Claims 1 to 16.

21. Composition according to Claim 20, **characterized in that** the concentration of at least one compound according to one of Claims 1 to 16 is between 0.001% and 5% by weight relative to the total weight of the composition.

22. Cosmetic composition, **characterized in that** it contains, in a cosmetically acceptable support, at least one compound as defined according to any one of Claims 1 to 16.

23. Composition according to Claim 22, **characterized in that** the concentration of at least one compound according to any one of Claims 1 to 16 is between 0.001 and 3% by weight relative to the total weight of the composition.

24. Use of a cosmetic composition as defined according to either of Claims 21 and 23, for body or hair hygiene.
